# EUROPEAN PATENT APPLICATION

(11) **EP 4 778 926 A1**
(43) Date of publication of application: **22.07.2026**
(21) Application number: 24864468.4
(22) Date of filing: 29.08.2024
(51) Int. Cl.: C07D 487/22, A61K 31/4188, A61P 21/02

(54) **DEUTERATED DIARYL GLYCOLURIL TETRAMER COMPOUND AND USE THEREOF**

(30) Priority: 13.09.2023 CN 202311180443
(71) Applicant: Shanghai Institute of Organic Chemistry, Chinese Academy of Sciences, Shanghai 200032 (CN)
(72) Inventor: LI, Zhanting, Shanghai 200032 (CN); YU, Shangbo, Shanghai 200032 (CN); FENG, Ke, Shanghai 200032 (CN); CAO, Jin, Shanghai 200032 (CN); ZONG, Yang, Shanghai 200032 (CN); LEI, Zhuo, Shanghai 200032 (CN); GUO, Peng, Shanghai 200032 (CN); TIAN, Jia, Shanghai 200032 (CN); QI, Qiaoyan, Shanghai 200032 (CN)
(74) Representative: Mewburn Ellis LLP
(86) International application number: PCT/CN2024/115569
(87) International publication number: WO 2025/055746

(57) **Abstract**

Disclosed are a deuterated diaryl glycoluril tetramer compound having aryls incorporated at both ends, as shown in formula (I), and a pharmaceutical composition containing the compound. The deuterated compound can be used for antagonizing a clinically used non-depolarizing muscle relaxant. At the same dosage, the deuterated compound has significantly better antagonistic activity against rocuronium bromide and vicuronium bromide than sugammadex sodium, and has significantly better antagonistic activity against cisatracurium besylate and rocuronium bromide than a non-deuterated control molecule.

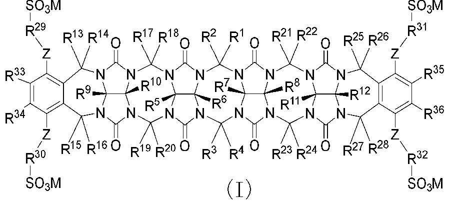

## Description

### Technical field

The present invention belongs to the field of medicine, specifically relates to a deuterated diaryl glycoluril tetramer compound or its combination that is capable of antagonizing various muscle relaxants.

### Background

Since 1942, neuromuscular blocking agents or skeletal muscle relaxants (hereafter referred to muscle relaxants for short) are widely used in clinical anesthesia practice to relax skeletal muscles, thereby benefiting intubation and improving surgical conditions. Every year, over four hundred million patients worldwide receive muscle relaxants treatment during anesthesia in operating rooms and intensive care units. Among them, non-depolarizing muscle relaxants, for example such as aminosteroidal agents such as rocuronium bromide, vecuronium bromide, and pancuronium bromide and benzylisoquinolinium compounds such as cisatracurium besylate, are more widely used in clinical practice due to their reduced risk of adverse effects compared with than depolarizing muscle relaxants. Cisatracurium besylate, an intermediate-acting muscle relaxant, accounts for approximately 30% of the global neuromuscular blocking agent market and 70% of the domestic market in China, owing to its rapid onset, potent efficacy, swift recovery profile, absence of histamine release, minimal cardiovascular side effects, no accumulation in the body, non-toxic metabolites without neuromuscular activity, and independent of renal excretion. In addition, rocuronium bromide and vecuronium bromide, which belong to the same category of intermediate-acting muscle relaxant as cisbenzenesulfonated atracurium, account for about 50% of the global market, and about 30% of the domestic market in China, while the long-acting muscle relaxant pancuronium bromide accounts for about 1%, but its absolute number of cases remains high. However, postoperative "residual tubocurarine toxicity " caused by residual intermediate-to-long-acting muscle relaxants has high incidence rate in clinical practice, which leads to postoperative respiratory complications such as upper respiratory dysfunction, respiratory obstruction, and pulmonary respiratory dysfunction, and in severe cases, causes patient death. Therefore, it is of great clinical significance to quickly and efficiently antagonize the residual excessive muscle relaxant, which is not only conducive to reducing the postoperative risk of patients, but also conducive to early extubation and rehabilitation of patients, speeding up the turnover of operating room and reducing the operating costs.

A prospective, multicenter, and single blind observational study published in 2016 in China found that the total incidence of residual muscle relaxants during endotracheal tube removal was up to 57.8%. It is necessary to intervene artificially with antagonists to eliminate "residual tubocurarine toxicity" and help patients recover muscle movement function as soon as possible. In current clinical practice, muscle relaxant antagonists are divided into two categories: (1) using anticholinesterase drugs to inhibit acetylcholinesterase activity, and (2) using the direct capture principle to bind and isolate muscle relaxants. The representative of the first type of antagonist is neostigmine, which needs to be combined with adjuvants such as glycopyrrolate or atropine to reduce the side effects of muscarine. The antagonistic efficiency of neostigmine is generally low, making it impossible to achieve rapid reversal of muscle relaxants and reversal of deep muscle relaxation. Therefore, Neostigmine is not an ideal muscle relaxant antagonist. The clinical practice representative drug of the second type of antagonist is sugammadex sodium, which can rapidly reverse the muscle relaxant activity of rocuronium and vecuronium through its specific binding to γ-cyclodextrin hydrophobic internal acupoints. However, sugammadex sodium cannot antagonize cisatracurium besylate and pancuronium bromide. Cisatracurium besylate is widely used in clinical practice and the most commonly used muscle relaxant in China, and is irreplaceable in many clinical practices: patients at risk of acute respiratory distress syndrome are recommended to use cisatracurium besylate as a surgical muscle relaxant, patients with renal insufficiency are recommended to use it as a general anesthesia drug. Cisatracurium besylate is one of the most commonly used muscle relaxants in intensive care units. Pancuronium bromide is still widely used as a long-acting muscle relaxant in clinical practice. Therefore, there is an unsolved important and urgent need in clinical practice to develop fast acting antagonists for cisatracurium besylate and pancuronium bromide, or broad-spectrum fast acting antagonists for all aforementioned muscle relaxants.

Hoffmann et al. reported that diphenyl glycouril tetramers with four sulfonate side chains have high biological safety, but low activity in antagonizing muscle relaxants. Liu et al. reported that the cucurbit [8]uril macrocycle introduced with sulfonate side chains has good activity in antagonizing muscle relaxants and biosafety. However, these cucurbit [8]uril derivatives are mixtures of uncertain ingredients and have low druggability. Xue et al. reported that the pillararene [6] hydrocarbon sulfonate has a high binding ability to cisatracurium besylate, but its actual antagonistic activity is unknown, and the synthesis yield of pillararene[6]hydrocarbon is low, and the separation of such kind of sulfonates also requires complicated gel chromatography technology. Therefore, although the development of broad-spectrum antagonistic drugs that can rapidly reverse muscle relaxants is beneficial in clinical practice, discovery of novel compounds with rapid antagonistic activity and high biosafety and having drugability is a very challenging task.

In summary, there is an urgent need in this field to develop a broad-spectrum antagonistic drug with high biosafety that can rapidly antagonize steroid quaternary ammonium salts and tetrahydroisoquinoline quaternary ammonium salt muscle relaxants.

### Summary of the invention

The purpose of the present invention is to provide a deuterated diaryl glycoluril tetramer compound with broad-spectrum rapid antagonistic activity against muscle relaxants such as steroidal quaternary ammonium salts (rocuronium bromide, vicuronium bromide, pancuronium bromide), and tetrahydroisoquinolinium quaternary ammonium salts (cisatracurium besylate), and its use.

In the first aspect of the present invention, provided is a deuterated diaryl glycoluril tetramer compound of Formula I, or a pharmaceutically acceptable salt, a hydrate, or a solvate thereof, 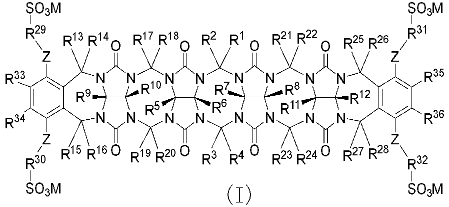 wherein,
each Z is independently O, S, Se, Te, C1-C4 alkylene, -NH- or amino substituted with C1-C4 alkyl;
each M is independently Na⁺, K⁺, Li⁺, Mg²⁺, Ca²⁺, NH₄⁺, or ammonium salts substituted with one or more C1-C4 alkyl;
R¹, R², R³, R⁴, R⁵, R⁶, R⁷, R⁸, R¹³, R¹⁴, R¹⁵, R¹⁶, R¹⁷, R¹⁸, R¹⁹, R²⁰, R²¹, R²², R²³, R²⁴, R²⁵, R²⁶, R²⁷, and R²⁸ are each independently deuterium or hydrogen;
R⁹, R¹⁰, R¹¹, and R¹² are each independently non-deuterated, partially deuterated or fully deuterated linear or branched C1-C20 alkyl, or R⁹ and R¹⁰, R¹¹ and R¹² together with the attached carbon atom each form a non-deuterated, partially deuterated or fully deuterated group selected from the group consisting of: C3-C20 cycloalkyl, 3-20 membered heterocyclyl, C5-C20 aryl, and 5-20 membered heteroaryl;
R²⁹, R³⁰, R³¹, and R³² are each independently non-deuterated, partially deuterated, or fully deuterated groups selected from the group consisting of: linear or branched C1-C20 alkylene, C3-C20 cycloalkylene, linear or branched 1-20 membered heteroalkylene containing one or more heteroatoms selected from oxygen, sulfur, and nitrogen, and 3-20 membered heterocyclylene containing one or more heteroatoms selected from oxygen, sulfur, and nitrogen;
R³³, R³⁴, R³⁵, and R³⁶ are each independently deuterium, hydrogen, or non-deuterated, partially deuterated, or fully deuterated groups selected from the group consisting of: linear or branched C1-C20 alkyl, C3-C20 cycloalkyl, linear or branched C1-C20 alkoxy, linear or branched C1-C20 alkylthio, linear or branched C1-C20 aldehyde group, linear or branched C1-C20 ester group, linear or branched C1-C20 alkyl containing carbonyl, or R³³ and R³⁴, R³⁵ and R³⁶ together with the attached carbon atom each form a non-deuterated, partially deuterated, or fully deuterated group selected from the group consisting of: C3-C20 saturated carbocycle, C3-C20 saturated spiral-ring, 3-20 membered saturated heterocycle containing one or more heteroatoms selected from oxygen, sulfur, and nitrogen, C5-C20 aromatic ring, and 5-20 membered heteroaromatic ring containing one or more heteroatoms selected from oxygen, sulfur, and nitrogen;
with the proviso that: at least one of R¹, R², R³, R⁴, R⁵, R⁶, R⁷, R⁸, R¹³, R¹⁴, R¹⁵, R¹⁶, R¹⁷, R¹⁸, R¹⁹, R²⁰, R²¹, R²², R²³, R²⁴, R²⁵, R²⁶, R²⁷, R²⁸, R³³, R³⁴, R³⁵, and R³⁶ is deuterium, or at least one of R⁹, R¹⁰, R¹¹, R¹², R²⁹, R³⁰, R³¹, R³², R³³, R³⁴, R³⁵, and R³⁶ is deuterium or deuterated.

In another preferred embodiment, Z is O, S, Se, Te, methylene, NH, or amino substituted with C1-C4 alkyl.

In another preferred embodiment, M is Na⁺.

In another preferred embodiment, R¹, R², R³, and R⁴ are each independently H or D.

In another preferred embodiment, at least one of R¹, R², R³, and R⁴ is D.

In another preferred embodiment, R¹, R², R³, and R⁴ are each independently D.

In another preferred embodiment, R⁵, R⁶, R⁷ and R⁸ are each independently deuterium or hydrogen.

In another preferred embodiment, R⁵, R⁶, R⁷ and R⁸ are each independently hydrogen.

In another preferred embodiment, R¹³, R¹⁴, R¹⁵, R¹⁶, R¹⁷, R¹⁸, R¹⁹, R²⁰, R²¹, R²², R²³, R²⁴, R²⁵, R²⁶, R²⁷, and R²⁸ are each independently H or D.

In another preferred embodiment, at least one of R¹³, R¹⁴, R¹⁵, R¹⁶, R¹⁷, R¹⁸, R¹⁹, R²⁰, R²¹, R²², R²³, R²⁴, R²⁵, R²⁶, R²⁷, and R²⁸ is D.

In another preferred embodiment, R¹³, R¹⁴, R¹⁵, R¹⁶, R¹⁷, R¹⁸, R¹⁹, R²⁰, R²¹, R²², R²³, R²⁴, R²⁵, R²⁶, R²⁷, and R²⁸ are each independently D.

In another preferred embodiment, R¹³, R¹⁴, R¹⁵, R¹⁶, R¹⁷, R¹⁸, R¹⁹, R²⁰, R²¹, R²², R²³, R²⁴, R²⁵, R²⁶, R²⁷, and R²⁸ are each independently H.

In another preferred embodiment, R¹, R², R³, R⁴, R¹⁴, R¹⁵, R¹⁶, R¹⁷, R¹⁸, R¹⁹, R²⁰, R²¹, R²², R²³, and R²⁴ are each independently H or D.

In another preferred embodiment, at least one, two, four, six, eight, or ten of R¹, R², R³, R⁴, R¹⁷, R¹⁸, R¹⁹, R²⁰, R²¹, R²², R²³, and R²⁴ are D.

In another preferred embodiment, R¹, R², R³, R⁴, R¹⁷, R¹⁸, R¹⁹, R²⁰, R²¹, R²², R²³, and R²⁴ are each independently D.

In another preferred embodiment, R⁹, R¹⁰, R¹¹, and R¹² are each independently non-deuterated, partially deuterated or fully deuterated linear or branched C1-C10 alkyl, or R⁹ and R¹⁰, R¹¹ and R¹² together with the attached carbon atom each form a group selected from the group consisting of: C3-C10 cycloalkyl, 3-10 membered heterocyclyl, C6-C10 aryl, and 5-10 membered heteroaryl.

In another preferred embodiment, R⁹, R¹⁰, R¹¹, and R¹² are each independently non-deuterated, partially deuterated or fully deuterated linear or branched C1-C7 alkyl, or R⁹ and R¹⁰, R¹¹ and R¹² together with the attached carbon atom each form C3-C7 cycloalkyl, 3-7 membered heterocyclyl, C6-C8 aryl, and 5-7 membered heteroaryl.

In another preferred embodiment, R⁹, R¹⁰, R¹¹, and R¹² are each independently non-deuterated, partially deuterated or fully deuterated linear or branched C1-C4 alkyl, or R⁹ and R¹⁰, R¹¹ and R¹² together with the attached carbon atom each form C5-C7 cycloalkyl.

In another preferred embodiment, R⁹, R¹⁰, R¹¹, and R¹² are each independently non-deuterated, partially deuterated or fully deuterated linear or branched C1-C4 alkyl.

In another preferred embodiment, R⁹, R¹⁰, R¹¹, and R¹² are each independently CD₃ or CH₃.

In another preferred embodiment, R²⁹, R³⁰, R³¹, and R³² are each independently non-deuterated, partially deuterated or fully deuterated groups selected from the group consisting of: linear or branched C1-C10 alkylene, C3-C10 cycloalkylene, linear or branched 1-10 membered heteroalkylene containing one or more heteroatoms selected from oxygen, sulfur, and nitrogen, and 3-10 membered heterocyclylene containing one or more heteroatoms selected from oxygen, sulfur, and nitrogen.

In another preferred embodiment, R²⁹, R³⁰, R³¹, and R³² are each independently non-deuterated, partially deuterated or fully deuterated groups selected from the group consisting of: linear or branched C1-C6 alkyl, and linear or branched 1-6 membered alkyl containing one or more heteroatoms selected from oxygen, sulfur, and nitrogen.

In another preferred embodiment, R²⁹, R³⁰, R³¹, and R³² are each independently non-deuterated, partially deuterated or fully deuterated groups selected from the group consisting of: linear or branched C1-C4 alkylene, and linear or branched 1-4 membered heteroalkylene containing one or more heteroatoms selected from oxygen, sulfur, and nitrogen.

In another preferred embodiment, R²⁹, R³⁰, R³¹, or R³² are each independently non-deuterated, partially deuterated or fully deuterated propyl.

In another preferred embodiment, R³³, R³⁴, R³⁵, and R³⁶ are each independently deuterium, hydrogen, or non-deuterated, partially deuterated, or fully deuterated groups selected from the group consisting of: linear or branched C1-C10 alkyl, C3-C10 cycloalkyl, linear or branched C1-C10 alkoxy, linear or branched C1-C10 alkylthio, linear or branched C1-C10 aldehyde group, linear or branched C1-C10 ester group, linear or branched C1-C10 alkyl containing carbonyl, or R³³ and R³⁴, R³⁵ and R³⁶ together with the attached carbon atom each form a non-deuterated, partially deuterated, or fully deuterated group selected from the group consisting of: C3-C10 saturated carbocycle, C3-C10 saturated spiral-ring, 3-10 membered saturated heterocycle containing one or more heteroatoms selected from oxygen, sulfur, and nitrogen, C5-C10 aromatic ring, and 5-10 membered heteroaromatic ring containing one or more heteroatoms selected from oxygen, sulfur, and nitrogen.

In another preferred embodiment, R³³, R³⁴, R³⁵, and R³⁶ are each independently deuterium, hydrogen, or non-deuterated, partially deuterated, or fully deuterated groups selected from the group consisting of: linear or branched C1-C6 alkyl, C3-C7 cycloalkyl, linear or branched C1-C6 alkoxy, linear or branched C1-C6 alkylthio, linear or branched C1-C6 aldehyde group, linear or branched C1-6 ester group, linear or branched C1-C6 alkyl containing carbonyl, or R³³ and R³⁴, R³⁵ and R³⁶ together with the attached carbon atom each form a non-deuterated, partially deuterated, or fully deuterated group selected from the group consisting of: C3-C7 saturated carbocycle, C3-C7 saturated spiral-ring, 3-7 membered saturated heterocycle containing one or more heteroatoms selected from oxygen, sulfur, and nitrogen, C6-C10 aromatic ring, and 5-8 membered heteroaromatic ring containing one or more heteroatoms selected from oxygen, sulfur, and nitrogen.

In another preferred embodiment, R³³, R³⁴, R³⁵, and R³⁶ are each independently deuterium, hydrogen, or non-deuterated, partially deuterated, or fully deuterated groups selected from the group consisting of: linear or branched C1-C6 alkyl, C3-C7 cycloalkyl, or R³³ and R³⁴, R³⁵ and R³⁶ together with the attached carbon atom each form a non-deuterated, partially deuterated, or fully deuterated group selected from the group consisting of: C3-C7 saturated carbocycle and benzene ring.

In another preferred embodiment, R³³, R³⁴, R³⁵, and R³⁶ are each independently deuterium, hydrogen, or non-deuterated, partially deuterated, or fully deuterated groups selected from the group consisting of: linear or branched C1-C5 alkyl, or R³³ and R³⁴, R³⁵ and R³⁶ together with the attached carbon atom each form a non-deuterated, partially deuterated, or fully deuterated group selected from the group consisting of: C5-C7 saturated carbocycle and benzene ring.

In another preferred embodiment, R³³, R³⁴, R³⁵, and R³⁶ are each independently deuterium, hydrogen, CD₃, or CH₃, or R³³ and R³⁴, R³⁵ and R³⁶ together with the attached carbon atom each form 5-membered carbocycle and benzene ring.

In another preferred embodiment, the ratio of deuterium atoms to the sum of deuterium atoms and hydrogen atoms (D/(D+H)) in the compound is ≥ 0.015% (natural deuterium isotope content); preferably, ≥ 10%; more preferably, ≥ 30%; more preferably, ≥ 75%; more preferably, ≥ 95%; most preferably, ≥ 98%.

In another preferred embodiment, the compound includes at least one deuterium atom; preferably, 2 deuterium atoms; more preferably, 4 deuterium atoms; more preferably, 6 deuterium atoms; more preferably, 8 deuterium atoms; more preferably, 12 deuterium atoms; most preferably, 20 deuterium atoms.

In another preferred embodiment, the compound does not include non-deuterated or deuterium-free compounds.

In another preferred embodiment, the compound is selected from the group consisting of:

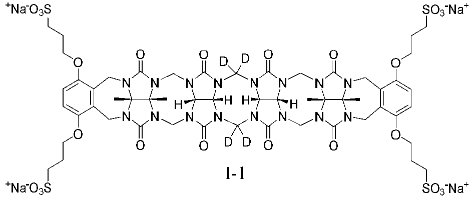

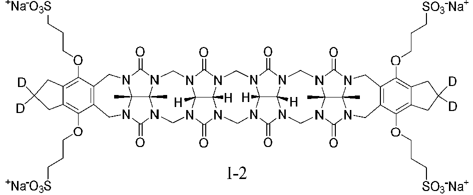

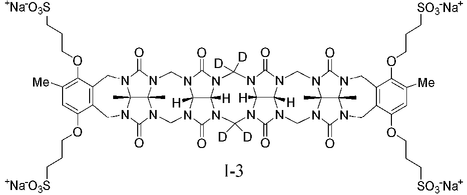

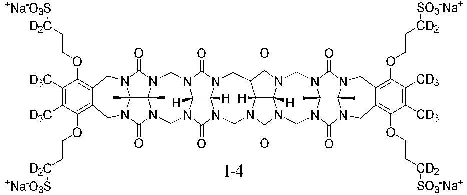

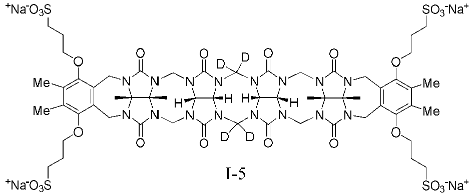

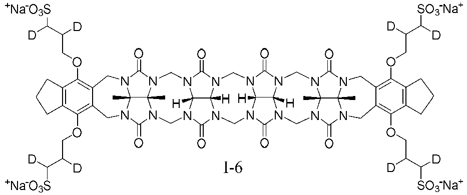

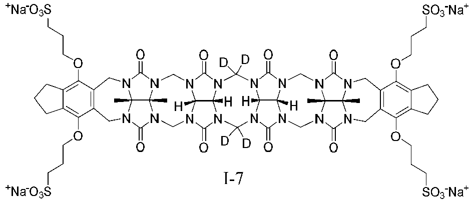

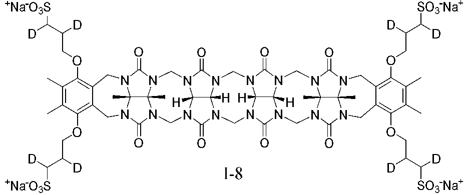

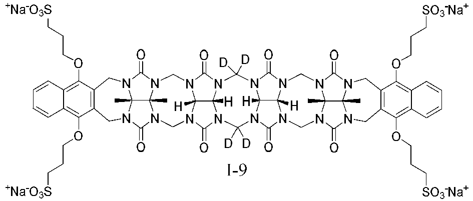

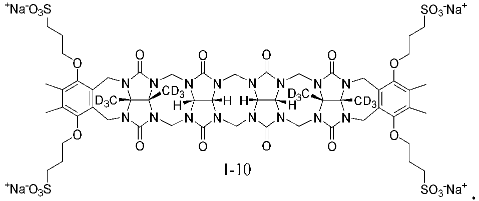

In another preferred embodiment, the compound is

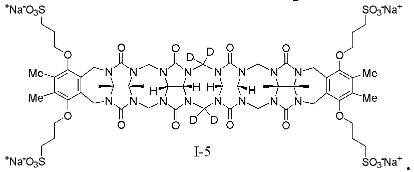

In the second aspect of the present invention, provided is a pharmaceutical composition, comprising (a) the compound according to the first aspect of the present invention, or a pharmaceutically acceptable salt, a hydrate or a solvate thereof as an active ingredient; and (b) pharmaceutically acceptable carriers.

In another preferred embodiment, component (a) accounts for 0.001-99.999 wt% of the total weight of the composition; preferably 0.01-99.99 wt%; more preferably 0.1-90 wt%.

In another preferred embodiment, the dosage form of the pharmaceutical composition is injection, tablets, capsules, pills, suspension, or emulsion.

In another preferred embodiment, the injection is an aqueous solution, sodium chloride aqueous solution, or glucose aqueous solution, wherein the concentration of the compound according to the first aspect of the present invention as the active ingredient is 0.1-5000 mg/mL.

In another preferred embodiment, the dosage of the injection is 0.1-5000 mg/kg, based on the body weight of rats.

In the third aspect of the present invention, provided is a pharmaceutical composition, comprising:
(a) the compound according to first aspect of the present invention, or a pharmaceutically acceptable salt, a hydrate or a solvate thereof as a first active ingredient;
(b) an ingredient with antagonistic effects on non-depolarizing muscle relaxants as a second active ingredient, and is selected from the group consisting of: Neostigmine, Sugammadex Sodium, or a combination thereof; and
(c) pharmaceutically acceptable carriers or excipients.

In another preferred embodiment, the dosage form of the pharmaceutical composition is injection, tablets, capsules, pills, suspension, or emulsion.

In the fourth aspect of the present invention, provided is a pharmaceutical composition, comprising:
(a) the compound according to the first aspect of the present invention, or a pharmaceutically acceptable salt, a hydrate or a solvate thereof as a first active ingredient;
(b) non-deuterated aryl tetramer compound with the structure shown in the first aspect of the present invention as a second active ingredient; and
(c) pharmaceutically acceptable carriers or excipients.

In another preferred embodiment, the dosage form of the pharmaceutical composition is injection, tablets, capsules, pills, suspension, or emulsion.

In another preferred embodiment, the non-deuterated aryl tetramer compound having the structure shown in the first aspect of the present invention is as follows: 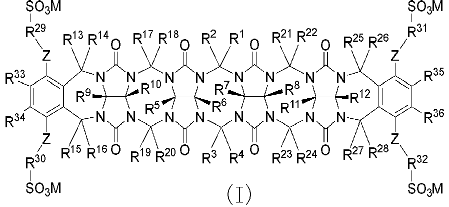
wherein, each Z is independently O, S, Se, Te, C1-C4 alkylene, -NH-, amino substituted with C1-C4 alkyl;
each M is independently Na⁺, K⁺, Li⁺, Mg²⁺, Ca²⁺, NH₄⁺, or ammonium salts substituted with one or more C1-C4 alkyl;
R¹, R², R³, R⁴, R⁵, R⁶, R⁷, R⁸, R¹³, R¹⁴, R¹⁵, R¹⁶, R¹⁷, R¹⁸, R¹⁹, R²⁰, R²¹, R²², R²³, R²⁴, R²⁵, R²⁶, R²⁷, and R²⁸ are each independently hydrogen;
R⁹, R¹⁰, R¹¹, and R¹² are each independently non-deuterated linear or branched C1-C20 alkyl, or R⁹ and R¹⁰, R¹¹ and R¹² each together with the attached carbon atom form a non-deuterated group selected from the group consisting of: C3-C20 cycloalkyl, 3-20 membered heterocyclyl, C5-C20 aryl, and 5-20 membered heteroaryl;
R²⁹, R³⁰, R³¹, and R³² are each independently non-deuterated groups selected from the group consisting of: linear or branched C1-C20 alkylene, C3-C20 cycloalkylene, linear or branched 1-20 membered heteroalkylene containing one or more heteroatoms selected from oxygen, sulfur, and nitrogen, and 3-20 membered heterocyclylene containing one or more heteroatoms selected from oxygen, sulfur, and nitrogen;
R³³, R³⁴, R³⁵, and R³⁶ are each independently hydrogen, or non-deuterated groups selected from the group consisting of: linear or branched C1-C20 alkyl, C3-C20 cycloalkyl, linear or branched C1-C20 alkoxy, linear or branched C1-C20 alkylthio, linear or branched C1-C20 aldehyde group, linear or branched C1-C20 ester group, linear or branched C1-C20 alkyl containing carbonyl, or R³³ and R³⁴, R³⁵ and R³⁶ each together with the attached carbon atom form a non-deuterated group selected from the group consisting of: C3-C20 saturated carbocycle, C3-C20 saturated spiral-ring, 3-20 membered saturated heterocycle containing one or more heteroatoms selected from oxygen, sulfur, and nitrogen, C5-C20 aromatic ring, and 5-20 membered heteroaromatic ring containing one or more heteroatoms selected from oxygen, sulfur, and nitrogen.

In the fifth aspect of the present invention, provided is a kit, comprising:
(a) a first container, and the compound according to the first aspect of the present invention, or a pharmaceutically acceptable salt, a hydrate, or a solvate thereof or the pharmaceutical composition according to the second aspect of the present invention as the active ingredient placed in the first container; and/or
(b) a second container, and the muscle relaxant placed in the second container; the muscle relaxant is selected from the group consisting of: steroid quaternary ammonium salts, tetrahydroisoquinoline quaternary ammonium salts, benzylisoquinoline derivatives, or combinations thereof; and/or
(c) a n-th container, and the n-th drug component placed in the n-th container, wherein n is any positive integer from 3 to 30; wherein, the n-th drug component is selected from the compound according to the first aspect of the present invention; and/or
(4) optional instructions.

In another preferred embodiment, the non-depolarizing muscle relaxant is cisatracurium besylate, pancuronium bromide, rocuronium bromide, vecuronium bromide, or a combination thereof.

In another preferred embodiment, the kit is used to antagonize muscle relaxants.

In another preferred embodiment, the non-depolarizing muscle relaxant is selected from the group consisting of: steroid quaternary ammonium salts, tetrahydroisoquinoline quaternary ammonium salts, benzylisoquinoline derivatives muscle relaxant, or combinations thereof;

In the sixth aspect of the present invention, provided is a use of the compound according to the first aspect of the present invention, the pharmaceutical composition according to the second aspect of the present invention, or the pharmaceutical composition according to the third aspect of the present invention or the pharmaceutical composition according to the fourth aspect of the present invention for antagonizing a muscle relaxant.

In the seventh aspect of the present invention, provided is a method for antagonizing muscle relaxants by administering a therapeutically effective amount of the compound according to the first aspect of the present invention, or a pharmaceutically acceptable salt, a hydrate, or a solvate thereof, to a subject in need thereof, thereby antagonizing residual tubocurarine toxicity in the subject's body.

It should be understood that, within the scope of the present invention, each of the above technical features of the present invention and each of the technical features specifically described in the following (such as the embodiments) can be combined with each other to constitute a new or preferred technical solution. Due to space limitations, It will not be repeated herein.

### EMBODIMENTS FOR CARRYING OUT THE INVENTION

Through extensive, in-depth, and systematic research, the inventor has developed a class of deuterated diaryl glycoluril tetramer compound with broad-spectrum rapid antagonistic activity against muscle relaxants such as steroidal quaternary ammonium salts (rocuronium bromide, vicuronium bromide, pancuronium bromide), and tetrahydroisoquinolinium quaternary ammonium salts (cisatracurium besylate). These compounds also have high water solubility and biological safety. At the same dose, the deuterated compound of the present invention exhibits significantly enhanced antagonistic activity compared to non-deuterated control compound and sugammadex sodium. The inventor has completed the present invention on this basis.

### Definition of groups

Unless otherwise defined, all technical and scientific terms used herein have the same meaning as would normally be understood by those of ordinary skill in the art to which the invention belongs.

As used herein, term "about" means that the value may change by no more than 1% from the enumerated value when used in reference to a specific enumerated value. For example, as used herein, the expression "about 100" includes 99 and 101 and values between 99 and 101 (e. g., 99.1, 99.2, 99.3, 99.4, etc.).

As used herein, term "contain" or "include (comprise)" may be open, semi-closed, and closed. In other words, said term also includes "consisting essentially of" or "consisting of".

Deuterium is a stable isotope of hydrogen. The C-D bond is more stable than the C-H bond, so the C-D bond is less prone to breakage compared to the C-H bond, and its half-life may be extended. The acidity and hydrophobicity of the C-D bond are different from those of the C-H bond, and their stacking, clustering, or aggregation driven by hydrophobic interactions in vivo, as well as their stability and selectivity in binding to living substances, may vary. The metabolic processes of living systems are complex, and the metabolism and excretion dynamics of deuterated compounds in living organisms are influenced by various factors, which are expected to exhibit corresponding complexity. Therefore, compared to non-deuterated compounds, the biological activity of deuterated compounds exhibits great randomness and unpredictability, and deuterium substituton at many sites may reduce or degrade the biological activity of the compound. In addition, due to limitations in synthesis methods, hydrogen in certain position of the organic compound is difficult or hard to be deuterated. Therefore, the deuterium substitution of organic compound is not arbitrary, and the sites for deuterium substitution are unpredictable, and their biological activity is also unpredictable.

As used herein, "C1-C20 alkyl" represents a linear or branched chain alkyl having 1 to 20 carbon atoms, such as methyl, ethyl, propyl, isopropyl, N-butyl, isobutyl, tert-butyl and the like.

As used herein, "1-20 membered heteroalkyl" refers to a group where one or more carbon atom in the chain of C₁₋₂₀ alkyl is replaced by heteroatoms selected from nitrogen, oxygen, and sulfur. For example, 1-8 membered heteroalkyl refers to CH₃-CH₂-CH₂-CH₂-O-CH₂-CH₂-CH₂-, or CH₃-CH₂-CH₂-CH₂-O-CH₂-CH₂-O- and the like.

As used herein, "2-20-membered heteroalkenyl" refers to a group where one or more carbon atoms in the chain of C₂₋₂₀ alkenyl are replaced by heteroatoms selected from nitrogen, oxygen, and sulfur. For example, 1-7-membered heteroalkenyl refers to groups such as CH₃-CH₂-CH₂-O-CH₂-CH=CH-, or CH₃-S-CH₂-CH₂-O-CH=CH-, and the like.

As used herein, "2-20-membered heteroalkynyl" refers to a group where one or more carbon atoms in the chain of C₂₋₂₀ alkynyl are replaced by heteroatoms selected from nitrogen, oxygen, and sulfur. For example, 1-7-membered heteroalkynyl refers to groups such as CH₃-CH₂-CH₂-O-CH₂-C≡C-, or CH₃-S-CH₂-CH₂-O-C≡C-, and the like.

As used herein, "C1-C20 alkyl chain" represents a linear or branched chain alkyl chain (CH₂)ₙ(n = 1~20).

As used herein, "C3-C20 cycloalkyl" refers to a cyclic alkyl group or a cyclic alkyl group with side chains that contains 3-20 carbon atoms, such as cyclopropyl, cyclobutyl, cyclopentyl, cyclohexyl, etc. The term "C₃-C₂₀" refers to a cycloalkyl containing 3, 4, 5, 6, 7, 8, 9, 10, 11, 12, 13, 14, 15, 16, 17, 18, 19, or 20 carbon atoms. The preferred cycloalkyl is C₃-C₁₄ cycloalkyl, more preferably C₃-C₁₀ cycloalkyl, more preferably C₃-C₆ monocyclic cycloalkyl, C₇-C₁₀ bicyclic or tricyclic cycloalkyl. "Substituted cycloalkyl" refers to a cycloalkyl having substitutions at one or more positions, especially having 1-4 substituents, which can be substituted at any position.

As used herein, "carbocycle" refers to a fully saturated or partially unsaturated (preferably fully saturated) cyclic hydrocarbon compound group, including cycloalkenyl, cycloalkyl, and cycloalkynyl.

In the present invention, the term "heterocyclyl (or heterocycle)" refers to a fully saturated or partially unsaturated cyclic group (including but not limited to for example 3-7 membered monocyclic, 4-7 membered monocyclic, 6-11 membered bicyclic, or 8-16 membered tricyclic or polycyclic system), wherein at least one heteroatom is present in a ring containing at least one carbon atom. The term "4-20 membered heterocyclyl" refers to a heterocycy containing 4, 5, 6, 7, 8, 9, 10, 11, 12, 13, 14, 15, 16, 17, 18, 19, or 20 ring atoms. "Heterocyclyl" and "saturated or unsaturated heterocyclyl" have the same meaning. The "heterocyclyl" is preferably a 4-14 membered heterocyclyl (including but not limited to such as 4-6 membered monocyclic, 7-10 membered bicyclic, or 8-14 membered tricyclic or polycyclic system), more preferably a 4-12 membered heterocyclyl, more preferably a 4-10 membered heterocyclyl such as 4-6 membered monocyclic heterocyclyl, 7-11 membered bicyclic or tricyclic heterocyclyl, more preferably a 4-8 membered heterocyclyl, and more preferably a 4-6 membered heterocyclyl. The heterocyclic ring containing heteroatoms in each heterocyclyl can have 1, 2, 3, or 4 heteroatoms independently selected from nitrogen, oxygen, and sulfur atoms, wherein nitrogen or sulfur atoms can be oxidized and nitrogen atoms can also be quaternized. Heterocyclyl can be attached to any residue of heteroatoms or carbon atoms in the ring or ring system molecule, preferably to N or C atoms in the ring or ring system molecule. Typical monocyclic heterocycles include, but are not limited to, azetidinyl, pyrrolidinyl, oxetanyl, pyrazolinyl, imidazolinyl, imidazolidinyl, oxazolidinyl, isoxazolidinyl, thiazolidinyl, isothiazolidinyl, tetrahydrofuryl, piperidyl, piperazinyl, 2-oxopiperazinyl, 2-oxopiperidyl, 2-oxopyrrolidinyl, hexahydroazepinyl, 4-piperidonyl, tetrahydropyranyl, morpholinyl, thiomorpholinyl, thiomorpholinyl sulfoxide, thiomorpholinyl sulfinyl, 1,3-dioxanyl, and 1,1-dioxothiolane. Polycyclic heterocyclyl includes spirocyclic, fused cyclic, and bridged heterocyclic groups; wherein, the heterocyclic groups involved in the spiral, fused, and bridged rings can optionally be connected to other groups through single bonds, or further connected to other cycloalkyl, heterocyclyl, aryl, and heteroaryl through any two or more atoms on the ring; "heterocyclyl" may be substituted or unsubstituted. When substituted, the substituent may be one or more of the following groups, and independently selected from the group consisting of: alkyl, deuterated alkyl, halogenated alkyl, alkoxyl, halogenated alkoxyl, alkenyl, alkenyl, alkylthiol, alkylamino, halogen, amino, nitro, hydroxyl, thiol, cyano, cycloalkyl, heterocyclyl, aryl, heteroaryl, cycloalkylthiol, oxo, carboxyl, and carboxylate, etc..

As used herein, "deuterated" refers to one or more hydrogen atoms in a compound or group being replaced by deuterium. Deuteration can be mono substituted, di-substituted, poly-substituted, or fully substituted. The terms "one or more deuterated" and "one or more deuteration" can be used interchangeably.

As used herein, "non-deuterated" refers to the deuterium isotopic abundance at each hydrogen position not exceeding the natural isotope content (approximately 0.015%).

As used herein, the term "active ingredient" refers to deuterated diaryl glycoluril tetramer compound. It should be understood that the term also include mixtures of such compounds.

As used herein, the term "hydrate" refers to a complex formed by the coordination of the compound of the present invention with water.

As used herein, the term "solvate" refers to a complexe formed by the coordination of the compound of the present invention and solvent molecules at a specific ratio.

As used herein, the term "aryl (aromatic ring)" refers to monovalent aromatic carbocyclyl having from 5 to 20 (preferably from 6 to 14) carbon atoms, which has a monocyclic ring (such as phenyl) or fused ring (such as naphthyl or anthryl). If the connecting point is located on the aromatic carbon atom, the fused ring may be non-aromatic (such as 2-benzoxazolone, 2H-1,4-benzoxazin-3(4H)-one-7-yl, etc.). Preferably, aryl includes phenyl and naphthyl.

As used herein, the term "cycloalkyl" refers to a cyclic alkyl having 3-12 carbon atoms and monocyclic and polycyclic ring (including fused, bridged and spiro ring system). In a fused ring system, one or more rings can be cycloalkyl, heterocyclyl, aryl, or heteroaryl, as long as the connecting site is through a ring of cycloalkyl. Examples of suitable cycloalkyl include: for example, adamantyl, cyclopropyl, cyclobutyl, cyclopentyl, and cyclooctyl.

The term "heteroaryl" refers to an aromatic cyclic hydrocarbon group having 1 -4 heteroatoms, wherein the heteroatom is selected from oxygen, nitrogen, and sulfur. Wherein, the "5-14 membered heteroaryl" refers to a heteroaromatic system having 1 to 4 heteroatoms and 5 to 14 ring atoms. The heteroaryl is either monocyclic (such as pyridyl or furyl) or fused cyclic (such as indolizinyl or benzothienyl). Among them, the fused ring may be non-aromatic and/or having one heteroatom, as long as the connecting point is through an aromatic heteroaromatic atom. Heteroaryl is preferably 5 to 10 membered ring, and more preferably 5 or 6 membered ring, such as pyrrolyl, pyrazolyl, imidazolyl, oxazolyl, isoxazolyl, thiazolyl, thiadiazolyl, isothiazolyl, furyl, pyridinyl, pyrazinyl, pyrimidinyl, pyridazinyl, triazinyl, triazolyl, and tetrazolyl. "Heteroaryl" may be substituted or unsubstituted. When substituted, the substituent may be one or more of the following groups, and independently selected from the group consisting of: alkyl, deuterated alkyl, halogenated alkyl, alkoxyl, halogenated alkoxyl, alkenyl, alkenyl, alkylthiol, alkylamino, halogen, amino, nitro, hydroxyl, thiol, cyano, cycloalkyl, heterocyclyl, aryl, heteroaryl, cycloalkylthiol, oxo, carboxyl, and carboxylate, etc.. In one embodiment, the ring atom nitrogen and/or sulfur of the heteroaryl is optionally oxidized to N-oxide (N-O), sulfonyl or sulfonyl. Preferably heteroaryl includes pyridinyl, pyrrolyl, indolyl, thienyl, and furyl.

As used herein, the term "substituted heteroaryl" refers to a heteroaryl substituted with from 1 to 5, preferably from 1 to 3, and more preferably from 1 to 2 substituents, wherein the substituent is the same as those defined in the substituted aryl.

The animal activity test and acute toxicity test in the present invention use SD rats for relevant tests.

As used herein, unless otherwise specified, the "substituted" refers to the substitution of hydrogens by one or more groups selected from the group consisting of: halogen, nitro, amino, cyano, hydroxyl, amido, trifluoromethyl, C₁₋₆alkyl, C₂₋₆alkenyl, C₂₋₆alkynyl, C₁₋₆alkoxy, phenyl, benzyl, 3-6 membered heterocyclyl, and C₁₋₆alkoxycarbonyl.

As used herein, in diaryl glycoluril tetramer compounds, the chiral carbon atom, if exist, may be in the R configuration, the S configuration or a mixture of both.

The diaryl glycoluril tetramer compound of the present invention or combinations thereof may be prepared by methods well known to those skilled in the art in the prior art, and there are no particular limitations on the reaction parameters of the individual steps. In addition, typical compounds of the present invention are commercially available.

As used herein, "tubocurarine toxicity" refers to a postoperative condition in which patients fail to regain spontaneous breathing due to residual neuromuscular blockade caused by residual muscle relaxants. It is called "tubocurarine toxicity" because its symptoms resemble the use of tubocurarine.

The animal activity test and acute toxicity test in the present invention use SD rats for relevant tests.

### Neuromuscular Blocking Agents or muscle relaxants

Neuromuscular blocking agents or skeletal muscle relaxants (also known as muscle relaxants) are widely used in clinical practice to relax skeletal muscles, thereby benefiting intubation, or promoting surgical conditions. Muscle relaxants are categorized into depolarizing and non-depolarizing types based on their different mechanisms of action: depolarizing muscle relaxants bind to N2 cholinocepters on the motor nerve end plate membrane, weakening or disappearing the response of muscle cells to acetylcholine; non-depolarizing muscle relaxants compete with acetylcholine for N2 cholinocepters on the motor end plate membrane of skeletal muscle, leading to muscle relaxation. The non-depolarizing muscle relaxants commonly used in clinical practice are classified into amino steroid derivatives (such as rocuronium bromide, vecuronium bromide, and pancuronium bromide) and benzylisoquinoline derivatives (such as cisatracurium besylate) based on their chemical structures.

### Muscle relaxant antagonist - deuterated diaryl glycoluril tetramer

The present invention provides a deuterated diaryl glycoluril tetramer represented by formula I. 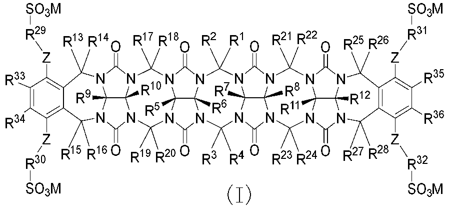 wherein, R¹, R², R³, R⁴, R⁵, R⁶, R⁷, R⁸, R⁹, R¹⁰, R¹¹, R¹², R¹³, R¹⁴, R¹⁵, R¹⁶, R¹⁷, R¹⁸, R¹⁹, R²⁰, R²¹, R²², R²³, R²⁴, R²⁵, R²⁶, R²⁷, R²⁸, R²⁹ , R³⁰, R³¹, and R³² are as defined above.

The deuterated diaryl glycoluril tetramer described in the present invention has a pre-organized "C" conformation, which forms a hydrophobic internal cavity that be drived to bind the aforementioned muscle relaxant through hydrophobic action, thereby antagonizing the muscle relaxation activity.

The deuterated diaryl glycoluril tetramer described in the present invention introduces negative ion sulfonate side chains at both ends, and its electrostatic interaction can exert a synergistic effect with the hydrophobic interaction of the "C" conformation of the tetramer, thereby rapidly and efficiently binding to muscle relaxants and antagonizing their muscle relaxation activity.

In the present invention, deuterated diaryl glycoluril tetramer is used as a broad-spectrum muscle relaxant antagonist. Rat experiments support that this application can achieve rapid antagonism of cisatracurium besylate and rocuronium bromide, as well as rapid antagonism of vecuronium bromide and pancuronium bromide muscle relaxants. This type of deuterated compound possesses high water solubility and high biological safety, and has significant clinical practicality.

Control experiments demonstrate that this type of new deuterated compounds can achieve faster antagonism against rocuronium bromide and vecuronium bromide than the clinically used drug sugammadex sodium. The control experiment with non-deuterated compounds supports that this type of deuterated compounds significantly enhance their antagonistic activity against cisatracurium besylate. Deuterated diaryl glycoluril tetramer is used as a broad-spectrum muscle relaxant antagonist, compared to their non-deuterated counterparts. Rat experiment supports that this application can achieve rapid antagonism of cisatracurium besylate and pancuronium bromide. Control experiments demonstrate that this type of new deuterated compounds can achieve faster antagonism against rocuronium bromide and vecuronium bromide than the clinically used drug sugammadex sodium. This type of deuterated compound possesses high water solubility and high biological safety, and has significant clinical practicality.

### Preparation method

The preparation method of compound of formula (I) of the present invention will be further described in the following and these specific methods do not constitute any limitation on the present invention. The compound of the present invention can also be conveniently prepared by combining various synthesis methods described in this specification or known in the art, and such combinations can be easily carried out by those skilled in the art to which the present invention belongs.

The deuterated diaryl glycoluril tetramer described in the present invention is prepared by electrophilic substitution reaction with two aromatic ring molecules with different functional groups, using an open ring glycoluril tetramer as a rigid pre-organized skeleton.

The preparation of deuterated diaryl glycoluril tetramer and the corresponding raw material in the present invention are mostly carried out under acidic conditions, from room temperature to reflux temperature (such as 0-100°C, preferably 0-60°C). The reaction time is usually 0.1-60 hours, preferably 0.5-48 hours. The following general preparation route can be used to synthesize compounds having the structure of formula (I) of the present invention:

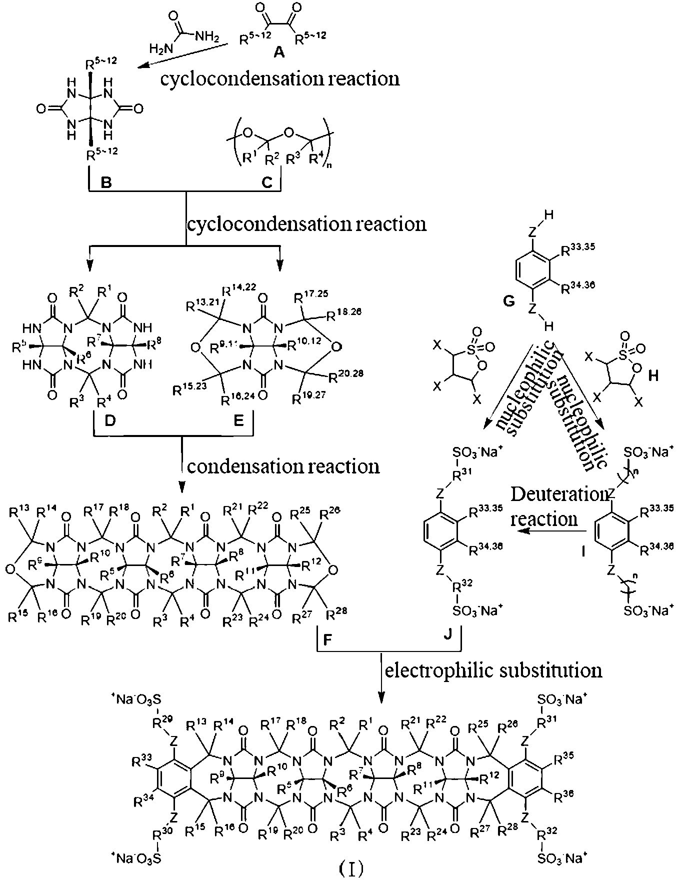

Synthesis route one: wherein Z is O, S, Se, Te, methylene, amino, and substituted amino;
R¹, R², R³, R⁴, R⁵, R⁶, R⁷, R⁸, R⁹, R¹⁰, R¹¹, R¹², R¹³, R¹⁴, R¹⁵, R¹⁶, R¹⁷, R¹⁸, R¹⁹, R²⁰, R²¹, R²², R²³, R²⁴, R²⁵, R²⁶, R²⁷, R²⁸, R²⁹ , R³⁰, R³¹, and R³² are as defined above.

As shown in synthesis route one, the compound of the present invention is synthesized using a convergent approach. (a) Firstly, diketone compound A with different substituents undergoes cyclocondensation reaction with urea to generate glycoluril compound B with different substituents. Subsequently, further cyclocondensation reaction is conducted with polyformaldehyde or deuterated polyformaldehyde C to generate glycoluril dimer D and tetracyclic glycoluril diether structure E, respectively. D and E undergo further condensation reaction to obtain tetramer F with different substituents; (b) The aryl compound G with different substituents undergoes nucleophilic substitution reaction with propane sultone H to generate non-deuterated aryl sulfonate I or J. I further undergoes deuteration reaction to obtain deuterated aryl sulfonate J. (c) Finally, non-deuterated or deuterated aryl sulfonate J aggregates with tetramer F to generate compound (I) of the present invention through electrophilic substitution reaction.

The deuterated diaryl glycoluril tetramer described in the present invention can be prepared using three key intermediates, namely deuterated or non-deuterated dimer intermediate, deuterated or non-deuterated tetramer intermediate, and deuterated or non-deuterated sulfonate intermediate. 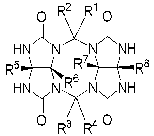 deuterated or non-deuterated dimer intermediate;
wherein, R¹, R², R³, R⁴, R⁵, R⁶, R⁷, and R⁸ are each independently deuterium or hydrogen;
furthermore, the structure of the deuterated or non-deuterated dimer intermediate is as follows: 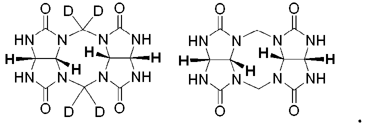 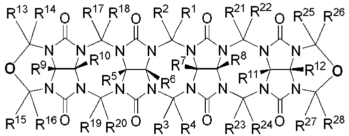 deuterated or non-deuterated tetramer intermediate;
wherein, R¹, R², R³, R⁴, R⁵, R⁶, R⁷, R⁸, R¹³, R¹⁴, R¹⁵, R¹⁶, R¹⁷, R¹⁸, R¹⁹, R²⁰, R²¹, R²², R²³, R²⁴, R²⁵, R²⁶, R²⁷, and R²⁸ are each independently deuterium or hydrogen; R⁹, R¹⁰, R¹¹, and R¹² are each independently non-deuterated, partially deuterated, or fully deuterated linear or branched C1-C20 alkyl, or two adjacent groups together with the attached carbon atom form non-deuterated, partially deuterated, or fully deuterated C3-C20 cycloalkyl;
further, the structure of the deuterated or non-deuterated tetramer intermediate is as follows: 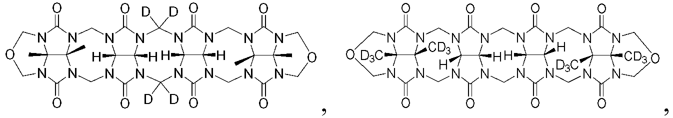 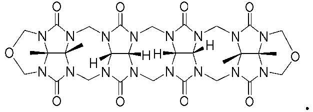 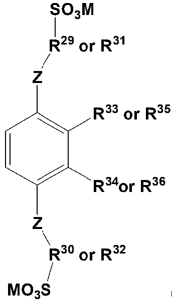 deuterated or non-deuterated tetramer sulfonate intermediate; wherein, Z is O, S, Se, Te, methylene, amino, or substituted amino; M is Na⁺;
R²⁹, R³⁰, R³¹, and R³² are each independently non-deuterated, partially deuterated, or fully deuterated groups selected from the group consisting of: linear or branched C1-C20 alkyl, C3-C20 cycloalkyl, linear or branched C1-C20 alkyl containing one or more heteroatoms selected from oxygen, sulfur, and nitrogen, and C3-C20 cycloalkyl containing one or more heteroatoms selected from oxygen, sulfur, and nitrogen;
R³³, R³⁴, R³⁵, and R³⁶ are each independently deuterium, hydrogen, or non-deuterated, partially deuterated, or fully deuterated groups selected from the group consisting of: linear or branched C1-C20 alkyl, C3-C20 cycloalkyl, linear or branched C1-C20 alkoxy, linear or branched C1-C20 alkylthio, linear or branched C1-C20 aldehyde group, linear or branched C1-C20 ester group, linear or branched C1-C20 alkyl containing carbonyl, or R³³ and R³⁴, R³⁵ and R³⁶ together with the attached carbon atom each form a non-deuterated, partially deuterated, or fully deuterated group selected from the group consisting of: C3-C20 saturated carbocycle, C3-C20 saturated spiral-ring, 3-20 membered saturated heterocycle containing one or more heteroatoms selected from oxygen, sulfur, and nitrogen, and C3-C20 aromatic ring;
furthermore, the sulfonate intermediate is selected from the group consisting of:

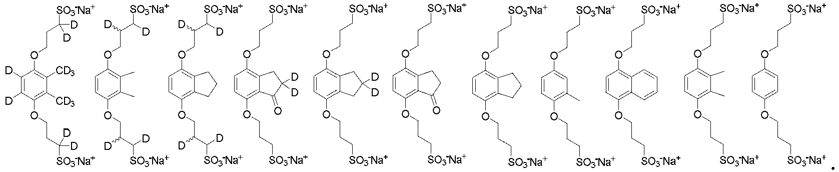

### Pharmaceutical composition

The pharmaceutical composition of the present invention comprises a safe and effective amount of the compound of the present invention, or a pharmaceutically acceptable salt thereof and pharmaceutically acceptable excipients or carriers. Wherein "safe and effective amount" refers to an amount of compound which is sufficient to significantly improve the condition, and not to generate severe side effects. Generally, the pharmaceutical composition contains 1-2000 mg of the compound of the present invention per dose, preferably, 10-1000mg the compound of the present invention per dose. Preferably, the "one dose" is one capsule or one pill.

As used herein, the "active ingredient" refers to the compound of formula I of the present invention, or a pharmaceutically acceptable salt, a hydrate or a solvate thereoof.

As used herein, the term "pharmaceutically acceptable salt" refers to non-toxic acid or alkaline earth metal salts of the compound of formula I. These salts can be prepared in situ during the final separation and purification of the compound of formula I, or by reacting suitable organic or inorganic acids or bases with basic or acidic functional groups, respectively. Representative salts include, but are not limited to: acetate, adipate, alginate, citrate, aspartate, benzoate, benzenesulfonate, bisulfate, butyrate, camphorate, camphorsulfonate, digluconate, cyclopentane propionate, dodecyl sulfate, ethane sulfonate, glucose enanthate, glycerophosphate, hemisulphate, enanthate, caproate, fumarate, hydrochloride, hydrobromide, hydroiodate, 2-hydroxyethanesulfonate, lactate, maleate, methane sulfonate, nicotinate, 2-naphthylsulfonate, oxalate, pamoate, pectate, thiocyanate, 3-phenylpropionate, picrate, pivalate, propionate, succinate, sulfate, tartrate, thiocyanate, p-toluenesulfonate, and undecanoate. In addition, nitrogen-containing basic group can be quaternized by the following reagents: alkyl halides, such as chlorides, bromides, and iodides of methyl, ethyl, propyl, and butyl; dialkyl sulfates, such as dimethyl, diethyl, dibutyl, and dipentyl sulfates; long chain halides such as chlorides, bromides, and iodides of decyl, lauryl, myristyl, and stearyl; arylalkyl halides such as benzyl and phenylethyl bromides. Thus, water-soluble, oil soluble, or dispersible products are obtained. Examples of acids that can be used to form pharmaceutically acceptable acid addition salts include inorganic acids such as hydrochloric acid, sulfuric acid, and phosphoric acid, as well as organic acids such as oxalic acid, maleic acid, methanesulfonic acid, succinic acid, and citric acid. Alkali addition salts can be prepared in situ during the final separation and purification of the compound of formula I, or by reacting the carboxylic acid moiety with suitable bases (such as pharmaceutically acceptable hydroxides, carbonates, or bicarbonates of metal cation) or ammonia, or organic primary, secondary, or tertiary amines. Pharmaceutically acceptable salts include, but are not limited to, cations based on alkali metals and alkaline earth metals such as sodium, lithium, potassium, calcium, magnesium, aluminum salts, as well as non-toxic ammonium, quaternary ammonium, and amine cations, including but not limited to: ammonium, tetramethylammonium, tetraethylammonium, methylamine, dimethylamine, trimethylamine, triethylamine, ethylamine, etc. Other representative organic amines used to form base addition salts include diethylamine, ethylenediamine, ethanolamine, diethanolamine, piperazine, etc.

"Pharmaceutically acceptable carrier" means one or more compatible solid or liquid fillers, or gelatinous materials which are suitable for human use and should be of sufficient purity and sufficiently low toxicity. "Compatible" herein refers to the ability of each component of a composition can be mixed with the compound of the present invention and can be mixed with each other without appreciably reducing the efficacy of the compound. Examples of pharmaceutically acceptable carrier include cellulose and derivatives thereof (such as sodium carboxymethylcellulose, sodium ethylcellulose, cellulose acetate, etc.), gelatin, talc, solid lubricant (such as stearic acid, magnesium stearate), calcium sulfate, vegetable oil (such as soybean oil, sesame oil, peanut oil, olive oil, etc.), polyol (such as propylene glycol, glycerol, mannitol, sorbitol, etc.), emulsifier (such as Tween^{®}), wetting agent (such as lauryl sodium sulfate), colorant, flavoring, stabilizer, antioxidant, preservative, pyrogen-free water, etc.

Suitable pharmaceutically acceptable carriers or excipients include: treatment agents and drug delivery modifiers and promoters, such as calcium phosphate, magnesium stearate, talc, monosaccharides, disaccharides, starch, gelatin, cellulose, sodium methylcellulose, carboxymethyl cellulose, glucose, hydroxypropyl-B-cyclodextrins, polyvinylpyrrolidone, low melting point wax, ion exchange resins, and any combination of two or more thereof. Liquid and semi-solid excipients can be selected from glycerol, propylene glycol, water, ethanol, and various oils, including petroleum, animal oil, vegetable oil, or synthetic sources, such as peanut oil, soybean oil, mineral oil, sesame oil, etc. Preferred liquid carriers, especially for injectable solutions, include water, saline, glucose aqueous solutions, and ethylene glycol. Other suitable pharmaceutically acceptable excipients are described in Remington's Pharmaceutical Sciences, Mack Pub. Co, New Jersey (1991), which is incorporated in this article by reference.

The pharmaceutical composition is injections, capsules, tablets, pills, powders or granules.

There is no special limitation of administration mode for the compound or pharmaceutical compositions of the present invention, and the representative administration mode includes (but is not limited to): oral, intratumoral, rectal, parenteral (intravenous, intramuscular or subcutaneous), and topical administration.

The solid dosage forms used for oral administration include capsules, tablets, pills, powders, and granules. In these solid dosage forms, the active compounds are mixed with at least one conventional inert excipient (or carrier), such as sodium citrate or dicalcium phosphate, or mixed with any of the following components: (a) fillers or compatibilizer, for example, starch, lactose, sucrose, glucose, mannitol and silicic acid; (b) binders, for example, hydroxymethyl cellulose, alginates, gelatin, polyvinylpyrrolidone, sucrose and arabic gum; (c) humectant, such as, glycerol; (d) disintegrating agents such as agar, calcium carbonate, potato starch or tapioca starch, alginic acid, certain composite silicates, and sodium carbonate; (e) dissolution-retarding agents, such as paraffin; (f) absorption accelerators, for example, quaternary ammonium compounds; (g) wetting agents, such as cetyl alcohol and glyceryl monostearate; (h) adsorbents, for example, kaolin; and (i) lubricants such as talc, stearin calcium, magnesium stearate, solid polyethylene glycol, sodium lauryl sulfate, or the mixtures thereof. In capsules, tablets, and pills, the dosage form may also include buffers.

Solid dosage forms such as tablets, sugar pills, capsules, pills, and granules can be prepared using coating and shell materials, such as casings and other materials well-known in the art. They may contain opacifiers, and the release of active compounds or compounds in the composition can be delayed in a certain part of the digestive tract. Examples of embedding components that may be employed are polymeric substances and waxes. If necessary, the active compound may also be formed into a microcapsules with one or more of the above excipients.

Liquid dosage forms for oral administration include pharmaceutically acceptable lotion, solutions, suspensions, syrups or tinctures. In addition to the active compounds, the liquid dosage forms may contain any conventional inert diluents known in the art such as water or other solvents, solubilizers and emulsifiers, for example, ethanol, isopropanol, ethyl carbonate, ethyl acetate, propylene glycol, 1,3-butanediol, dimethyl formamide, as well as oil, in particular, cottonseed oil, peanut oil, corn germ oil, olive oil, castor oil and sesame oil, or the combination thereof.

In addition to these inert diluents, the composition can also include additives such as wetting agents, emulsifiers and suspensions, sweeteners, flavoring agents, and spices.

In addition to active compounds, suspensions can include suspending agents such as ethoxylated isooctadecanol, polyoxyethylene sorbitol and dehydrated sorbitol esters, microcrystalline cellulose, methanol aluminum and agar, or mixtures of these substances.

Compositions for parenteral injection may include physiologically acceptable sterile aqueous or anhydrous solutions, dispersion liquid, suspensions or lotions, and sterile powders for re dissolution into sterile injectable solutions or dispersions. Suitable aqueous and non-aqueous carriers, diluents, solvents or excipients include water, ethanol, polyols, and their suitable mixtures.

The dosage forms of the compounds of the present invention used for local administration include ointments, powders, patches, sprays, and inhalants. The active ingredients are mixed under sterile conditions with physiologically acceptable carriers and any preservatives, buffers, and propellants if necessary.

The compound of the present invention may be administered alone or in combination with other pharmaceutically acceptable compound (such as antitumor drugs).

The treatment method of the present invention can be administered alone or in combination with other treatment methods or drugs.

When the pharmaceutical compositions are used, a safe and effective amount of compound of the present invention is applied to a mammal (such as human) in need of, wherein the dose of administration is a pharmaceutically effective dose. For a person weighed 60kg, the daily dose is usually 1- 2000mg, preferably 50-1000 mg. Of course, the particular dose should also depend on various factors, such as the route of administration, patient healthy status, which are within the skills of an experienced physician.

### Compared with prior art, the main advantages of the present invention include:

(1) Control experiments support that compared with the clinically used antagonist neostigmine, all the preferred examples of compounds of the present invention exhibit faster antagonism against both benzylisoquinoline muscle relaxant cisatracurium besylate and aminosteroid muscle relaxants pancuronium bromide, rocuronium bromide, and vecuronium bromide.
(2) Control experiments support that compared with the clinically used antagonist sugammadex sodium, the preferred examples of compounds of the present invention exhibit faster antagonism against aminosteroid muscle relaxants rocuronium bromide, and vecuronium bromide. The preferred examples of compounds of the present invention can rapidly antagonize the benzylisoquinoline muscle relaxant cisatracurium besylate and aminosteroid muscle relaxants pancuronium bromide, while sugammadex sodium can not antagonize these two types of muscle relaxants.
(3) Control experiments support that compared with the publicly available non-deuterated dibenzo-glycoluril tetramers, the preferred examples of compounds of the present invention has higher antagonistic activity against the four non-depolarizing muscle relaxants mentioned above, and also has high water solubility and high biological safety.
(4) Control experiments support that compared with the publicly available non-deuterated dimethyl-substituted dibenzo-glycoluril tetramers, the deuterated compounds of the present invention have a significant improvement on antagonistic activity against cisatracurium besylate and rocuronium bromide.

The present invention was further described hereafter in combination with specific embodiments. It should be understood that these examples are only used to illustrate the and not to limit the scope of the invention. The experimental methods with no specific conditions described in the following examples are generally performed under the conventional conditions, for example, according to J. Sambrook et al., Molecular Cloning: A Laboratory Manual, 3rd Edition, Science Press, 1989, or according to the manufacture's instructions. Unless otherwise stated, percentages and parts are by weight.

Unless otherwise defined, all professional and scientific terms used in the text have the same meanings as those familiar to those skilled in the art. In addition, any methods and materials similar or equal to the recorded content can be applied to the methods of the present invention. The preferred embodiments and materials described herein are for exemplary purposes only.

### Example 1: Synthesis of Compounds I-5

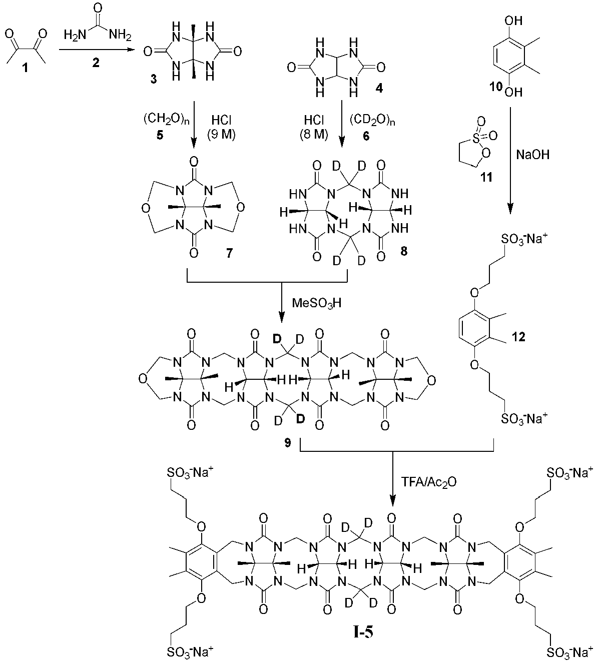

Under rapid mechanical stirring, hydrochloric acid (8 M, 80 mL) was added to a 500 mL double-necked flask, and glycoluril (compound 4, 49.3 g, 0.36 mol) was added in batches. The oil bath temperature was controlled at 50 °C. Subsequently, deuterated polyformaldehyde solution (compound 6, 10.7 g, 0.36 mol, dissolved in 40 mL of 8 M hydrochloric acid) was slowly added dropwise through a constant pressure dropping funnel. After the dropwise addition was complete, the mixture continued to be stirred for 48 hours (the reaction system was unclear and remained heterogeneous from start to finish). The reaction system was centrifugated, and the supernatant was transferred back to the double-necked flask; the solid was dispersed using 8M hydrochloric acid (2 mL) and added with deionized water (2 mL) to prepare to a 4M solution. The mixture was shook and centrifuged, and the upper yellow clear liquid was transferred to the aforementioned double-necked flask. After combined, the reaction continued at 50 °C for 48 hours. Subsequently, the "centrifugate - wash with 4 M hydrochloric acid - transfer and combine the supernatant" operation was repeated three times, and the final combined solid was dispersed with deionized water and then centrifugated. The " wash with deionized water - centrifugate" operation was repeated four times until the pH value of the supernatant was close to neutral. The white compound 8 was obtained (20.1 g, 37%). 1H NMR (400 MHz, DMSO-d6): δ 7.64 (s, 4H), 5.36 (d, J = 8.6 Hz, 2H), 5.23 (d, J = 8.7 Hz, 2H). 13C NMR (101 MHz, DMSO-d6): δ 158.38, 74.46, 60.63. HRMS (ESI): calcd for [M+H]+, 313.1306, found 313.1306.

Methylsulfonic acid (50 mL) was added to a 250 mL three-necked bottle, the temperature of the oil bath was raised to 50 °C, and then solid compound 8 (10.0 g, 32.0 mmol) was added in batches. The reaction system was stirred mechanically until the system was clear. Subsequently, solid compound 7 (24.4 g, 96.1 mmol) was added in batches and the reaction mixture was reacted at 50 °C for 5 hours under mechanical stirring. After the reaction was completed and cooled to room temperature, the reaction solution was added dropwise to water (500 mL) to produce a large amount of white precipitate. After centrifugation, the lower solid layer was collected and washed with deionized water (200 mL×5) until turing neutral, which was then transferred to a florence flask and dried in vacuum to obtain white solid compound 9 (15.0 g, 60%). 1H NMR (400 MHz, DMSO-d6) δ 5.54 - 5.45 (m, 6H), 5.34 (d, J = 9.1 Hz, 2H), 5.12 (d, J = 11.3 Hz, 4H), 4.79 (d, J = 10.7 Hz, 4H), 4.18 (d, J = 15.4 Hz, 4H), 1.76 (s, 6H), 1.59 (s, 6H). 13C NMR (101 MHz, DMSO-d6): δ 155.22, 154.67, 77.03, 72.29, 70.46, 70.03, 48.32, 17.63, 15.59. HRMS (ESI): calcd for [M+H]+,785.3125, found 785.3127.

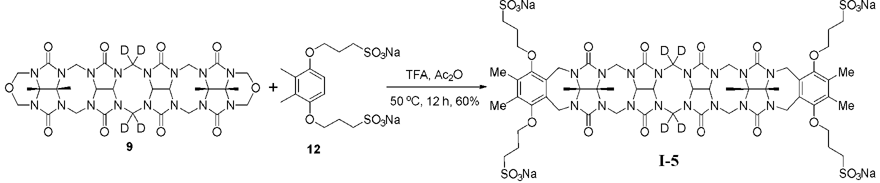

The dimer compound 9 (15.5 g, 19.8 mmol) was weighed and dissolved in trifluoroacetic acid (75 mL). Then acetic anhydride (75 mL) and **sodium 2,3-dimethyl-1,4-propane sulfonate** (compound 12, 23.6 g, 59.3 mmol) were added in sequence, and the reaction was carried out at 50 °C for 12 hours. After the reaction was complete, the reaction solution was added dropwise to rapidly stirred ethanol (1.3 L), stirred for half an hour, and centrifuged. The solids were washed with ethanol (30 mL × 5) in centrifuge tubes. After that, the solids were dissolved in water (20 mL), and filtered, and the solid residue obtained by spin-drying the filtrate was recrystallized with ethanol and water. The crystals were left overnight in a refrigerator at 4 ° C and filtered. The filter cake was washed with ethanol aqueous solution (5 mL, 4:1 v/v), and the obtained white solid was dissolved in water (6 mL) and filtered. The filtrate was evaporated to remove water at 70 ° C and dried in vacuum to obtain a white solid compound I-5 (19.1 g, 60%). 1H NMR (400 MHz, D2O): δ 5.42 (d, J = 15.7 Hz, 4H), 5.34-5.20 (m, 4H), 5.01 (d, J = 16.5 Hz, 4H), 4.22 (d, J = 16.2 Hz, 4H), 4.12 (d, J = 15.6 Hz, 4H), 3.88-3.76 (m, 4H), 3.67-3.54 (m, 4H), 3.02 (q, J = 7.0 Hz, 8H), 2.09 (t, J = 7.3 Hz, 8H), 1.73-1.56 (m, 24H). 13C NMR (101 MHz, D2O): δ 156.87, 156.23, 150.34, 131.58, 128.31, 78.69, 77.63, 72.81, 71.25, 48.56, 48.06, 36.31, 25.01, 16.52, 15.47, 12.60. HRMS (ESI): Calcd for [M+2Na]2+: 823.1707, Found: 823.1696.

### Example 2: Synthesis of Compound I -1

The method described in Example 1 was followed, except that sodium 1,4-phenoxy-dipropylalkane sulfonate was used instead of sodium 2,3-dimethyl-1,4-phenoxydipropylsulfonate.

The four-deuterated dimethyl glycoluril tetramer (1.0 g, 1.26 mmol) was weighed and dissolved in trifluoroacetic acid (6 mL). Then acetic anhydride (6 mL) and 1,4-phenoxy-dipropylalkane sulfonate (1.52 g, 3.82 mmol) were added in sequence, and the mixture was reacted at 50 °C for 12 hours. After the reaction was complete, the reaction solution was added dropwise to ethanol (200 L) that is under rapidly stirred, after stirring for half an hour, the mixture was centrifuged. The solids were washed with ethanol (30 mL × 5) in centrifuge tubes and centrifuged. After that, the solids were dissolved in water (20 mL), and filtered, and the solid residue obtained by spin-drying the filtrate was recrystallized with ethanol and water. The crystals were left overnight in a refrigerator at 4 ° C and filtered. The filter cake was washed with ethanol aqueous solution (5 mL, 4:1 v/v), and then recrystallized again with ethanol/water. The obtained white solid was dissolved in water (8 mL) and filtered. The filtrate was evaporated to remove water at 70 ° C and dried in vacuum to obtain a white solid compound I-1 (1.1 g, 56%). 1H NMR (400 MHz, D2O): δ 6.78 (s, 4H), 5.56 (d, J = 15.4 Hz, 4H), 5.46 -5.38 (m, 4H), 5.34 (d, J = 16.1 Hz, 4H), 4.26 (d, J = 15.3 Hz, 8H), 4.03-3.95 (m, 4H), 3.92-3.84 (m, 4H), 3.19-3.06 (m, 8H), 2.18 (d, J = 6.2 Hz, 8H), 1.77 (d, J = 15.6 Hz, 12H). 13C NMR (101 MHz, D2O): δ 156.75, 156.43, 150.12, 127.78, 114.94, 78.88, 77.69, 71.16, 71.01, 68.69, 48.48, 48.21, 35.26, 24.68, 16.46, 15.38. HRMS (ESI): Calcd for [M+2Na]2+: 795.1394, Found: 795.1382.

### Example 3: Synthesis of Compound I -2

The method described in Example 1 was followed, except that deuterated compounds 3-4 was used instead of sodium 2,3-dimethyl-1,4-phenoxydipropylsulfonate.

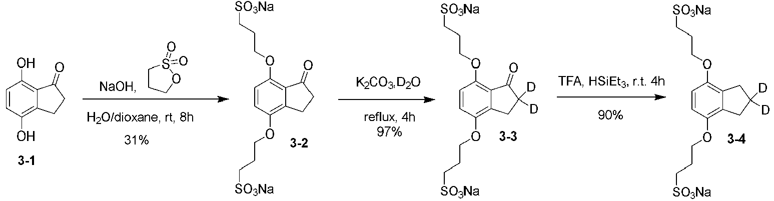

Compound 4,7-dihydroxy-1-indanone (synthesized according to literature methods, 820.0 mg 5 mmol) was weighed and added into a 50ml single-neck bottle. 500.9 mg (12.5 mmol) of NaOH was dissolved in 10.0ml of H₂O, and the mixture was added to the reaction system under stirring. The brown solid turned into a dark green liquid, and the reaction solution was stirred at room temperature for 30 minutes. 1.8330g (15 mmol) of 1,3-propanesultone was dissolved in 20.0ml of 1,4-dioxane and added to the system under stirring. The reaction was carried out overnight at room temperature, and the raw materials reacted completely. The reaction solution was concentrated and added dropwise to 100ml of EtOH resulting in the formation of brown precipitates. Centrifugation was carried out to afford a brown solid with a black surface. The obtained solid was washed twice with 60 mL of EtOH. The solid crude product was recrystallized using EtOH/H₂O at 90 °C to obtain a light brown solid powder 3-2 (701.2mg, 31%). 1H NMR (400 MHz, D2O) δ 7.26 (d, J = 8.8 Hz, 1H), 6.92 (d, J = 9.2 Hz, 1H), 4.19 (m, J = 6.4, 14.4 Hz, 4H), 3.07 (q, J = 8.0 Hz, 4H), 3.01(t, J = 5.6 Hz, 2H), 2.69 (t, J = 5.6 Hz, 2H), 2.21 (m, J = 12.0, 6.0 Hz, 4H). 13C NMR (101 MHz, D¬2O, 298K): δ 207.12, 148.26, 146.51, 144.58, 122.45, 117.68, 108.69, 64.93, 64.64, 45.76, 45.67, 34.28, 22.28, 21.90, 19.75. HRMS (ESI): cacld for [M-Na]-, 429.0295; found, 429.0323.

Sodium benzocyclopentanone sulfonate (452.2 mg, 1.0 mmol) was weighed and added into a 25ml single-neck flask, potassium carbonate (13.8 mg, 0.1 mmol) was added to 2ml of heavy water, and the mixture was heated to reflux for 4 hours. After the reaction was complete, the reaction system was poured into 20mL of ethanol, resulting in the formation of precipitates. Centrifugation and drying were carried out to afford light brown deuterated sodium benzocyclopentanone sulfonate (439.5mg, 97%). 1H NMR (400 MHz, D2O) δ 7.17 (d, J = 8.8 Hz, 1H), 6.83 (d, J = 8.8 Hz, 1H), 4.14 (m, J = 6.4, 14.0 Hz, 4H), 3.07 (m, J = 7.6, 15.2 Hz, 4H), 3.01(s, 2H), 2.17 (m, J = 9.6, 15.2 Hz, 4H). 13C NMR (101 MHz, D2O, 298 K) δ 210.03, 150.66, 148.89, 147.36, 125.06, 120.66, 111.27, 67.45, 66.94, 47.85, 47.74, 35.97, 24.36, 23.95, 21.88. HRMS (ESI) calcd: [M-Na]- 415.0415, found 415.0421; calcd: [M-2Na]2- 204.0264, found 204.0277.

Deuterated sodium benzocyclopentanone sulfonate (454.2 mg, 1.0 mmol) was added to a 50 mL single-neck flask, followed by the addition of TFA(1.14 g, 10 mmol). The solution turned orange red and was stirred at room temperature for 15 minutes. HSiEt3 (288.15 mg, 2.5 mmol) was slowly added dropwise to the above system. After reacting for 4 hours, the reaction system was completed and distilled under reduced pressure to remove unreacted TFA and HSiEt3. The resulting solid crude product was recrystallized using water and ethanol to afford a white powdery solid product of deuterated sodium benzocyclopentane sulfonate 3-4 (396.4 mg, 90%). 1H NMR (400 MHz, D2O) δ 6.80 (s, 1H), 4.09 (t, J = 6.0 Hz, 4H), 3.03 (m, J = 5.6, 8.0 Hz, 4H), 2.8 (s, 2H), 2.12 (m, J = 6.0, 14.0 Hz, 4H). M/Z = 197.0368. 13C NMR (101 MHz, D2O, 298 K) δ 149.24, 134.91, 112.46, 67.88, 47.98, 29.42, 29.33, 24.45. HRMS (ESI) calcd: [M -2Na]2-, 197.0368, found 197.0402; [M -2Na + H]-, 395.0809, found 395.0806.

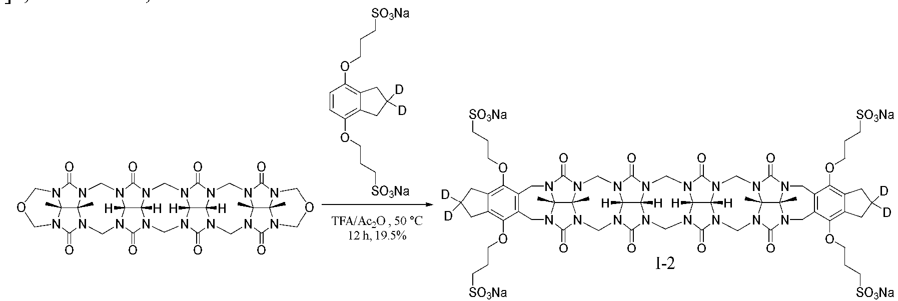

Methyl tetramer (189.0 mg, 0.25 mmol) and a mixed solvent of TFA/Ac₂O (2 mL/2 mL) were added successively into a 25 mL single-neck flask and stirred to dissolve. Subsequently, the system was added with deuterated benzocyclopentane sulfonate (286.0 mg, 0.625 mmol), and heated to 50 °C and reacted for 12 hours. After the reaction was complete, the reaction system was slowly added dropwise into 80 mL of ethanol under rapid stirring, resulting in the formation of precipitates. Centrifugation was carried out to afford the crude product. The crude product was recrystallized using a mixed solvent of EtOH/H₂O at 70 °C to afford the solid target product I-2 (80.2 mg, 19.5% ) as a white powder. 1H NMR (400 MHz, D2O) δ 5.64 (d, J = 15.2 Hz, 2H), 5.52 (d, J = 15.6 Hz, 4H), 5.34 (m, J = 8.8,31.2 Hz, 4H), 5.15 (d, J = 9.2 Hz, 4H), 4.31 (d, J = 16.0 Hz, 4H), 4.20 (d, J = 15.6 Hz, 4H), 4.02 (m, J = 10.0, 15.2 Hz, 6H), 3.81 (m, J = 6.0, 15.6 Hz, 8H), 3.08 (m, J = 18.0, 8.4 Hz, 8H), 2.45 (d, J =26.4, 16.0 Hz, 8H), 2.13 (m, J = 15.6, 8.8 Hz, 8H), 1.73 (d, J = 13.6 Hz, 12H). 13C NMR (101 MHz, D¬2O): δ 156.60, 156.12, 148.23, 138.11, 128.67, 78.68, 77.59, 71.78, 71.50, 71.10, 36.00, 30.17, 24.99, 17.28, 16.79, 15.82, 15.81. HRMS (ESI): Calcd for [M + Na +H]2+ : 842.1789; found: 824.1798.

### Example 4: Synthesis of Compound I -3

The method described in Example 1 was followed, except that compound 4-2 was used instead of sodium 2,3-dimethyl-1,4-phenoxydipropylsulfonate.

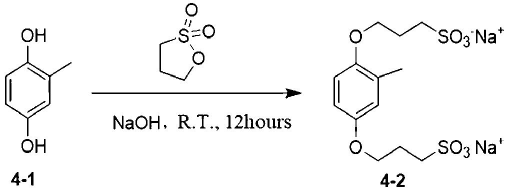

2,5-dihydroxytoluene (29.79 g, 10 mmol) was added to a 2 L reaction flask, followed by the addition of NaOH solution (36.92 g, 38.5 mmol, dissolved in 370 mL of deionized water). The light yellow solid turned into a dark green solution, and the reaction continued stirring at room temperature for 30 minutes. The reaction system was added dropwise with a solution of 1,3-propanesultone in dioxane (72.41 g, 24.7 mmol, dissolved in 60 mL of dioxane) and continued to react at room temperature for 12 hours. As the reaction proceeded, a large amount of precipitates appeared in the system, and the reaction system gradually turned from black to brown. After the reaction was complete, a light yellow solid was collected by filtration, and then washed successively with ethanol (50mL 2) and acetone (50mL 2). The resulting solid was recrystallized using water and ethanol at 90 °C, and dried in vacuum to afford a light yellow solid compound 4-2 (64.29 g, 73%). 1H NMR (400 MHz, D¬2O): δ 6.89 (d, J = 8.8 Hz, 1H), 6.80 (s, 1H), 6.75 (dd, J1= 8.8 Hz, J2= 2.8 Hz, 1H), 4.01 (t, J1= 7.2 Hz, 4H), 2.98 (m, 4H), 2.13-2.03 (m, 4H), 2.11 (s, 3H). 13C NMR (101 MHz, D¬2O): δ 149.21, 134.74, 112.25, 67.77, 48.00, 29.50, 24.47. HRMS (ESI): Calcd: [M-Na]-, 389.0346, Found: 389.0406.

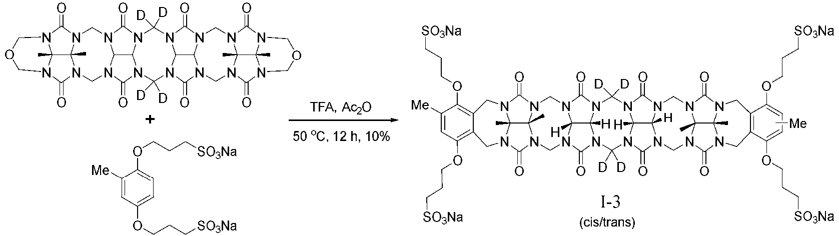

Methyl tetramer (1.0 g, 1.275 mmol) was added to a 50ml single-neck flask , followed by the addition of 5.0 mL of acetic anhydride and 5.0 ml of trifluoroacetic acid, and then stirred to dissolve. Compound 4-2 (1.5772 g, 3.825 mmol) was added to the reaction system, and then stirred to dissolve. The system was heated to 50 °C and reacted for 12 hours. After the reaction was completed, the reaction solution was added dropwise to 80 ml of ethanol, resulting in the formation of brown solid precipitates. After centrifugation, the lower precipitate layer was washed twice with 100 ml of ethanol, refluxed with MeOH for 3 hours, and then hot-filtered. The filter cake was taken and purified by diffusion with water/acetone and tetrahydrofuran system to obtain white solid product I-3 (200.8 mg, 10%). 1H NMR (400 MHz, D2O) δ 6.80 (d, J = 9.6 Hz, 2H), 5.60 (d, J = 15.7 Hz, 4H), 5.46 (d, J = 9.0 Hz, 2H), 5.41 (d, J = 9.0 Hz, 2H), 5.35 (dd, J = 16.3, 9.5 Hz, 2H), 5.21 (d, J = 5.8 Hz, 1H), 5.17 (d, J = 5.8 Hz, 1H), 4.37 (s, 1H), 4.34 (s, 1H), 4.28 (s, 2H), 4.24 (s, 3H), 4.21 (s, 1H), 4.17 (t, J = 8.4 Hz, 4H), 4.04 (d, J = 5.4 Hz, 3H), 3.94 (s, 2H), 3.22 - 3.11 (m, 8H), 2.29 - 2.20 (m, 8H), 2.15 (s, 6H), 1.82 (s, 6H), 1.77 (s, 6H). HRMS (ESI): Calcd: [M - 4Na + 2H]2-, 741.1947, Found: 741.1881.

### Example 5: Synthesis of Compound I-4

The method described in Example 1 was followed, except that compound 5-2 was used instead of sodium 2,3-dimethyl-1,4-phenoxydipropylsulfonate.

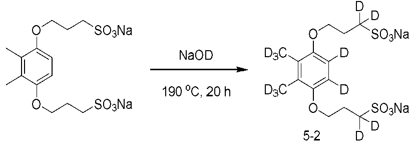

10 g of NaH was weighed and slowly added to 50 mL of heavy water, and stirred continuously. After the reaction was complete, n-hexane dried with anhydrous Na₂SO₄ was added, the residual paraffin oil was extracted, liquid separation was carried out three times to afford sodium deuteroxide solution. The high-pressure reactor was added with dimethyl phenoxydisulfonate (0.85 g, 2.0 mmol) and NaOD (100 mL), sealed, and then placed in an oil bath at 190 °C, and then stirred to react for 20 hours. After the reaction was complete and cooled to room temperature, the solids were filtered, collected, and washed with ethanol (2 mL 2). The resulting solid was dried in vacuum to afford a white solid 5-2 (0.71 g, yield 81%, deuterated rate 98%). 1H NMR (400 MHz, D2O): δ 4.05 (t, J = 6.4 Hz, 4H), 2.15 (t, J = 6.4 Hz, 4H).

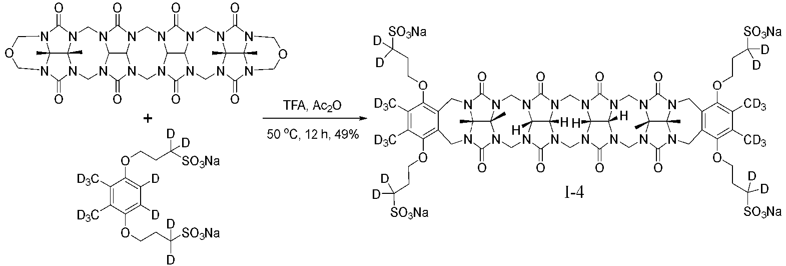

The four-deuterated dimethyl tetramer (0.418g, 0.5 mmol) was weighed and dissolved in trifluoroacetic acid (2 mL). Then acetic anhydride (2 mL) and twenty-deuterated sodium 2,3-dimethyl-1,4-phenoxydipropylsulfonate (0.65 g, 1.5 mmol) were added in sequence, and then the oil bath temperature was set at 50°C and the reaction system was reacted for 12h. After the reaction was complete, the reaction solution was added dropwise to ethanol (30 mL), stirred for 30 minutes, and then left stand and centrifuged. The solid was repeatedly washed with ethanol in a centrifuge tube (3 mL× 4) until the supernatant was essentially colorless. The solid was transferred to a 50 mL eggplant-shaped bottle to remove the solvent, and the resulting solid was recrystallized using ethanol and water. After filtration, the solid was washed with a small amount of a mixed solution of ethanol/water (4:1) and dried in vacuum to obtain white solid powder I-4 (0.398 g, 49%). 1H NMR (400 MHz, D2O): δ 5.64 (d, J = 15.4 Hz, 2H), 5.54 (d, J = 16 Hz, 4H), 5.42 (d, J = 9.2 Hz, 4H), 5.35 (d, J = 9.2 Hz, 4H), 5.13 (d, J = 16 Hz, 4H), 4.34 (d, J = 16 Hz, 4H), 4.24 (d, J = 16 Hz, 4H), 4.07 (d, J = 15.2 Hz, 2H), 3.91 (s, 4H), 3.70 (s, 4H), 2.17 (s, 4H), 1.74 (d, 12H). HRMS (ESI): Calcd: [M - 4Na + 2NH4]2-, 780.2871, Found: 780.4344.

### Example 6: Synthesis of Compound I -6

The method described in Example 1 was followed, except that compound 6-4 was used instead of sodium 2,3-dimethyl-1,4-phenoxydipropylsulfonate.

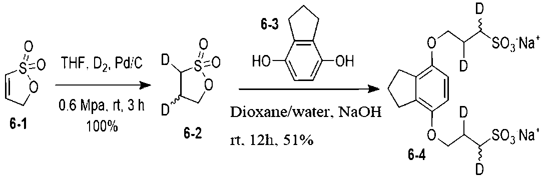

To a 100 ml thick-walled pressure resistant tube was added magneton and allyl-1,3-sultone (310.0 mg, 2.5 mmol), and then 50.0 ml of ether was added to dissolve under stirring. 10% Pd/C (110.3 mg) was added. The high-pressure reactor was evacuated and filled with deuterium gas to 0.6 MPa. The reaction started at room temperature and stopped when the pressure inside the reactor no longer changed. After the reaction was completed, palladium carbon was removed by filtering through diatomaceous earth, and washed three times with 10.0 ml of diethyl ether. The filtrate was evaporated to remove the solvent to obtain product as light yellow oily liquid, i.e., 1,2-dideuterated propane sultone 6-2 (0.31g, quantitative reaction), which did not require further purification and was directly used in the next step. ¹H NMR (400 MHz, CDCl₃) δ 4.43 (dd, *J =* 6.8, 1.6 Hz, 2H), 3.24 - 3.15 (m, 1H), 2.57 (dtd, *J* = 9.0, 6.9, 3.4 Hz, 1H). ¹³C NMR (101 MHz, CDCl₃) δ 77.61, 77.29, 76.97, 69.15, 69.06, 44.20, 44.12, 44.07, 43.94, 43.85, 43.71, 43.63, 23.64, 23.57, 23.45, 23.36, 23.24, 23.15. FIMS (FI) calcd for [M]: 124.0162, found 124.0161.

Compound 6-3 (754.4 mg, 5 mmol) was added to a 50 ml single-neck bottle. NaOH (508.8 mg, 12.5 mmol) was dissolved in 6.0 ml of H₂O and then added to the above system. The reaction system turned from a white solid to a dark green solution and was stirred at room temperature for 30 minutes; compound 6-2 (1.8624 g, 15 mmol) was dissolved in 12.0 mL of dioxane and added to the above system, and reacted at room temperature overnight. A large amount of brown precipitates generated during the reaction process, and the reaction system turned from a black solution to a brown suspension. After the reaction was complete, brown solids were collected by filtration, and then washed with a small amount of ethanol and acetone. The solids were transferred to a 50ml single-neck bottle and recrystallized with water and ethanol at 90 °C. Product **6-4** is beige powdery solid (1.2397 g , 60%) ¹H NMR (400 MHz, D₂O): δ 6.78 (s, 2H), 4.06 (d, *J =* 6.2 Hz, 4H), 3.01 (dd, *J =* 9.0, 6.7 Hz, 2H), 2.80 (t, *J =* 7.5 Hz, 4H), 2.22 - 2.05 (m, 2H), 2.04 (d, *J =* 7.5 Hz, 2H). ¹³C NMR (101 MHz, D2O) δ 149.17, 134.95, 112.51, 67.79, 47.78, 47.68, 47.48, 47.27, 29.44, 24.56, 24.12, 23.93, 23.74. HRMS ( ESI ) calcd for [M-2Na]²⁺: 198.0431, found 198.0427.

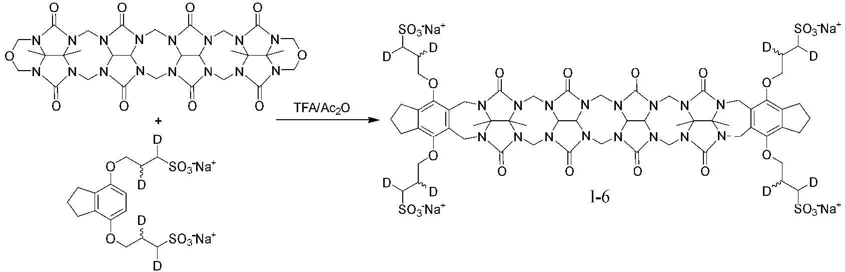

Methyl tetramer (782.3 mg, 1 mmol) was added to a 25ml single-neck flask, followed by the addition of 7.0 mL of acetic anhydride and 7.0 ml of trifluoroacetic acid, and then stirred to dissolve. Compound 6-4 (1.2397g, 3 mmol) was added to the reaction system, and then stirred to dissolve. The system was heated to 50 °C and reacted for 12 hours. After the reaction was completed, the reaction solution was added dropwise to 140 ml of ethanol, resulting in the formation of brown solid precipitates. After centrifugation, the lower precipitate layer was washed twice with 100 ml of ethanol. The lower solid was taken and recrystallized using water and ethanol at 70°C. After recrystallization, the product was spun-dried with an oil pump to afford a white powdery solid I-6 (0.6 g, 37%). ¹H NMR (500 MHz, D₂O) δ 5.66 (d, *J =* 15.3 Hz, 2H), 5.54 (d, *J =* 15.7 Hz, 4H), 5.46 - 5.33 (m, 4H), 5.16 (d, *J* = 15.9 Hz, 4H), 4.34 (d, *J =* 15.9 Hz, 4H), 4.24 (d, *J =* 15.6 Hz, 4H), 4.11 - 4.04 (m, 6H), 3.87 (d, *J= 8.2* Hz, 4H), 3.07 (dd, *J =* 19.3, 10.4 Hz, 4H), 2.51 (s, 8H), 2.13 (s, 4H), 1.75 (d, *J =* 24.7 Hz, 12H), 1.50 (s, 2H), 1.37 (s, 2H). ¹³C NMR (101 MHz, D₂O) δ 156.71, 156.15, 147.23, 138.15, 128.58, 78.63, 77.62, 71.76, 71.43, 71.09, 52.84, 48.45, 47.66, 36.03, 30.30, 25.57, 24.59, 17.23, 15.69. HRMS (ESI) calcd for [M-4Na+2H]²⁻: 769.2229, found 769.2243.

### Example 7: Synthesis of Compound I-7

The method described in Example 1 was followed, except that compound 7-4 was used instead of sodium 2,3-dimethyl-1,4-phenoxydipropylsulfonate.

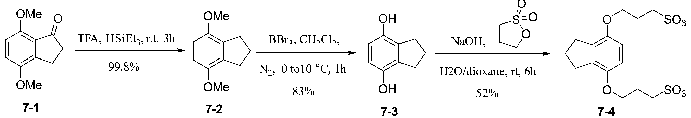

To a 250 mL single-neck flask was added 2,3-propylene-1,4-benzenediol (prepared according to the literature method, 2.07 g, 13.78 mmol), followed by the addition of NaOH aqueous solution (1.38 g, 34.45 mmol, dissolved in 37.5 mL of H₂O), and the mixture was stirred at room temperature for 30 minutes. Subsequently, a solution of 1,3-propanesultone in dioxane (5.05 g, 41.34 mmol, dissolved in 60 mL of 1,4-dioxane) was added to the system under stirring. The reaction was carried out at room temperature for 12 hours, and the raw materials reacted completely. After the reaction was completed, the reaction was centrifuged and the resulting solid was washed with EtOH (60 mL×2). The solid crude product was recrystallized using EtOH/H₂O at 90 °C to afford a light brown solid powder 7-4 (3.7520g, 70%). ¹H NMR (500 MHz, D₂O) δ 6.85 (s, 2H), 4.15 (t, *J =* 6.3 Hz, 4H), 3.14 - 3.00 (m, 4H), 2.88 (t, *J =* 7.4 Hz, 4H), 2.18 (dq, *J =* 12.6, 6.3 Hz, 4H), 2.08 (p, *J* = 7.7 Hz, 2H). ¹³C NMR (126 MHz, D₂O) δ 149.18, 134.92, 112.52, 67.89, 47.89, 29.45, 24.51, 24.36. HRMS (ESI) calcd: [M-Na]⁻, 415.0503, found 415.0501.

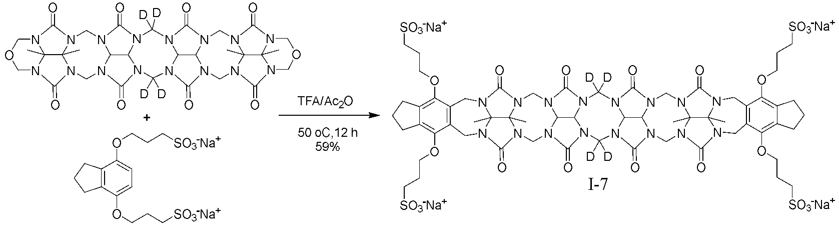

Four-deuterated dimethyl glycoluril tetramer (1.53 g, 1.79 mmol) and acetic anhydride (7.0 mL) were successively added to a 50 mL single-neck flask, and then stirred to dissolve. Trifluoroacetic acid (7.0 mL) and sodium 2,3-propylene-1,4-phenoxy-dipropylsulfonate (2.35 g, 5.35 mmol) were successively added to the reaction system, and stirred to dissolve. The system was heated to 50 °C and reacted for 12 hours. After the reaction was completed, the reaction solution was added dropwise to 140 mL of ethanol, resulting in the formation of light yellow solid precipitates. After centrifugation, the lower layer solids were washed twice with 150 mL of ethanol. The lower layer solids were taken and recrystallized using water and ethanol at 70°C. After recrystallization, the product was dried in vacuum to afford a white powdery solid I-7 (1.61g, 59%). ¹H NMR (500 MHz, D2O) δ 5.50 (d, *J =* 15.7 Hz, 4H), 5.38 (d, *J =* 9.1 Hz, 2H), 5.31 (d, *J* = 9.0 Hz, 2H), 5.12 (d, *J =* 16.0 Hz, 4H), 4.31 (d, *J =* 16.0 Hz, 4H), 4.19 (d, *J =* 15.7 Hz, 4H), 4.04 (q, *J =* 7.2 Hz, 4H), 3.82 (q, *J =* 7.9, 7.3 Hz, 4H), 3.05 (tt, *J =* 14.0, 6.8 Hz, 8H), 2.43 (d, *J =* 18.3 Hz, 8H), 2.23 - 2.03 (m, 8H), 1.71 (d, *J =* 21.8 Hz, 12H), 1.40 (s, 2H), 1.28 (s, 2H). ¹³C NMR (101 MHz, D₂O) δ 156.74, 156.20, 148.32, 138.21, 128.60, 78.67, 77.69, 71.89, 71.31, 71.17, 48.50, 48.12, 36.12, 30.36, 25.61, 25.03, 17.32, 15.74. HRMS (ESI): Calcd: [M - 3Na + H]2-, 778.2013, Found: 778.1961.

### Example 8: Synthesis of Compound I -8

The method described in Example 1 was followed, except that compound 8-2 was used instead of sodium 2,3-dimethyl-1,4-phenoxydipropylsulfonate.

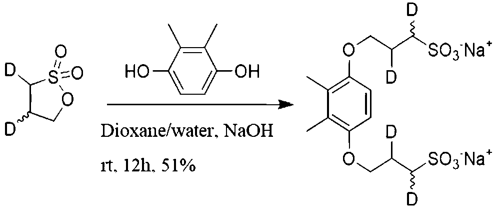

1,2-dideuterated propane sultone was prepared using the method described in Example 6. 3,4-Dimethylhydroquinone (0.35 g, 2.51 mmol) was added to a reaction flask, followed by the addition of 10% NaOH solution (0.25 g, 3.47 mmol, dissolved in 1.67 mL of deionized water). The light yellow solid turned into a dark green solution, and the reaction was continued to stir at room temperature for 30 minutes. The reaction system was added dropwise with a solution of 1,2-dideuterated propanesultone in dioxane (0.92 g, 7.53 mmol, dissolved in 4.2 L of dioxane) and continued to react at room temperature for 12 hours. As the reaction proceeded, a large amount of precipitates appeared in the system, and the reaction system gradually turned from black to brown. After the reaction was complete, a light yellow solid was collected by filtration, and then washed successively with ethanol (50mL ×2) and acetone (50mL ×2). The resulting solid was recrystallized using water and ethanol at 90 °C, and dried in vacuum to afford a light yellow solid compound 12 (0.55 g, 51 %). ¹H NMR (400 MHz, D₂O) δ 6.85 (s, 2H), 4.05 (d, *J* = 6.0 Hz, 4H), 3.08 (dd, *J₁ J₂ = J₁ J₂ =* 12.8 Hz, 2H), 2.14 (s, 6H). ¹³C NMR (101MHz, D₂O): δ 150.90, 128.12, 112.18, 68.47, 47.89, 47.68, 47.47, 24.30, 24.10, 23.9011.10. HRMS ( ESI ) calcd for [M-2Na]²⁻, 192.0516, found 192.0426; calcd for [M-2Na+H]⁻: 385.0994, found 385.0934; calcd for [M-Na]⁻, 407.0744, found 407.0754.

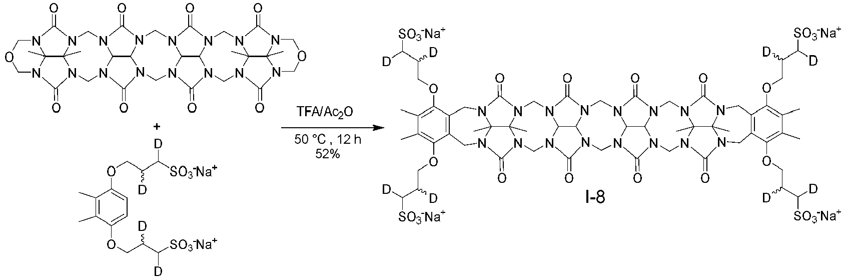

The four-deuterated dimethyl tetramer (0.32g, 0.387 mmol) was weighed and dissolved in trifluoroacetic acid (2 mL). Then acetic anhydride (2 mL) and four-deuterated sodium 2,3-dimethyl-1,4-phenoxydipropylsulfonate (0.5 g, 1.16 mmol) were added in sequence, and then the oil bath temperature was set at 50°C and the reaction system was reacted for 12h. After the reaction was complete, the reaction solution was added dropwise to ethanol (30 mL), stirred for 30 minutes, and then left stand and centrifuged. The solid was repeatedly washed with ethanol in a centrifuge tube (2 mL× 4) until the supernatant was essentially colorless. The solid was transferred to a 50 mL eggplant flask to remove the solvent, and the resulting solid was recrystallized using ethanol and water. After filtration, the solid was washed with a small amount of a mixed solution of ethanol/water (4:1) and dried in vacuum to obtain white solid powder I-8 (0.323 g, 52%). ¹H NMR (400 MHz, D₂O): δ 5.66 (d, *J =* 15.2 Hz, 2H), 5.57 (d, *J =* 15.2 Hz, 4H), 5.44 (d, *J* = 9.2 Hz, 4H), 5.37 (d, *J =* 15.2 Hz, 4H), 5.15 (d, *J =* 16 Hz, 4H), 4.34 (d, *J =* 16 Hz, 4H), 4.26 (d, *J =* 15.2 Hz, 4H), 4.09 (d, *J =* 15.2 Hz, 2H), 3.93 (s, 4H), 3.71 (s, 4H), 3.10 (s, 4H), 2.16 (s, 4H), 1.77 (s, 4H), 1.74 (s, 4H), 1.69 (s, 4H). HRMS (ESI): Calcd for [M - 3Na + H]2+:768.2138, Found: 768.2083.

### Example 9: Synthesis of Compound I -9

The method described in Example 1 was followed, except that sodium 1,4-naphthoxy-dipropylsulfonate was used instead of sodium 2,3-dimethyl-1,4-phenoxydipropylsulfonate.

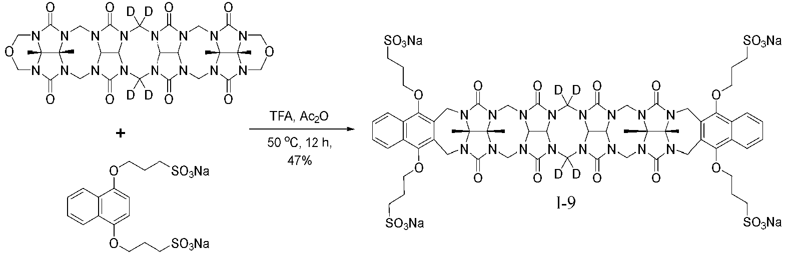

The four-deuterated dimethyl glycoluril tetramer (1.0 g, 1.26 mmol) was weighed and dissolved in trifluoroacetic acid (6 mL). Then acetic anhydride (6 mL) and sodium 1,4-naphthoxy-dipropylsulfonate (1.71 g, 3.50 mmol) were added in sequence, and the mixture was reacted at 50 °C for 12 hours. After the reaction was complete, the reaction solution was added dropwise to ethanol (200 L) that was under rapid stirring, and the mixture was stirred for half an hour, and then centrifuged. The solids were washed with ethanol (30 mL × 5) in a centrifuge tube. After that, the solids were dissolved in hot water (20 mL), and filtered, and the solid residue obtained by spin-drying the filtrate was recrystallized using ethanol and water. The crystals were left overnight in a refrigerator at 4 °C and filtered. The filter cake was washed with ethanol aqueous solution (5 mL, 4:1 v/v), and then recrystallized again with ethanol/water. The obtained white solid was dissolved in water (8 mL) and filtered. The filtrate was evaporated to remove water at 70 °C to obtain a white solid powder I-9 (0.98 g, 47%). 1H NMR (400 MHz, DMSO-d6): δ 7.78 (dd, J1 = 6.3, J2 = 3.1 Hz, 4H), 7.25 (dd, J1 = 6.1, J2 = 2.5 Hz, 4H), 5.58 (d, J = 15.2 Hz, 4H), 5.47 - 5.29 (m, 8H), 4.54 (d, J = 16.4 Hz, 4H), 4.25 (t, J = 12.9 Hz, 8H), 4.02 - 3.90 (m, 4H), 3.31 - 3.14 (m, 8H), 2.32 (d, J = 7.2 Hz, 8H), 1.84 (s, 12H). 13C NMR (101 MHz, D2O): δ 156.79, 156.37, 148.26, 127.76, 127.05, 126.15, 122.31, 78.68, 77.69, 74.25, 71.44, 71.28, 48.58, 48.06, 36.59, 25.17, 16.57, 15.37. HRMS (ESI): Calcd for [M+2Na]2+: 845.1551, Found: 845.1535.

### Example 10: Synthesis of Compound I -10

The method described in Example 1 was followed, except that hexadeuterated butanedione was used instead of non-deuterated butanedione; non-deuterated glycoluril dimer was used instead of deuterated glycoluril dimer.

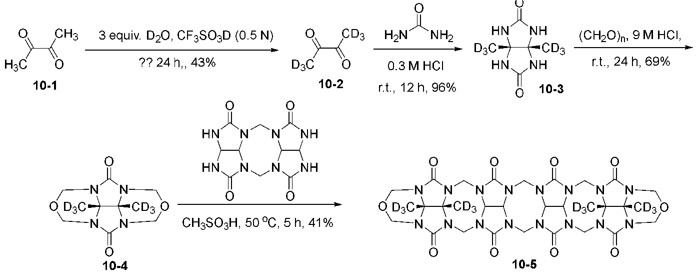

Urea (23.3 g, 0.388 mol) was weighed and dissolved in 0.3 M hydrochloric acid (56 mL). Deuterated butanedione (prepared according to the literature method, 10.8 g, 0.117 mol) was added slowly and dropwise under stirring, and the reaction was stirred at room temperature for 12 hours. After the reaction was completed, the reaction was filtered, the solid was washed with deionized water (10 mL×2) and dried in vacuum to afford a white solid of hexadeuterated dimethyl glycoluril (19.8 g, 96%). ¹H NMR (400 MHz, DMSO-*d*₆): δ 7.09 (s, 4H). ¹³C NMR (101 MHz, DMSO-d₆): δ 159.29, 74.98. HRMS (ESI): Calcd for [M+H]⁺: 177.1253, Found: 177.1250.

To a 250 mL eggplant shaped flask was weighed and added hexadeuterated dimethyl glycoluril (10.0 g, 56.8 mmol) and polyformaldehyde (8.6 g, 286.7 mmol), followed by the addition of 9 M hydrochloric acid (60 mL). The mixture was stirred at room temperature for 24 hours, and then added with deionized water (224 mL) and continued to stir for 12 hours. After the reaction was completed, the reaction was filtered. The collected solid was washed respectively with deionized water (50 mL×3) and ethanol (50 mL×3), and dried in vacuum to afford hexadeuterated dimethyl glycoluril diether (10.2 g, 69%) as a white solid (10.2 g, 69%). ¹H NMR (400 MHz, DMSO-d₆): δ 5.20 (d, *J =* 11.4 Hz, 4H), 4.99 (d, *J =* 11.4 Hz, 4H). ¹³C NMR (101 MHz, DMSO-d₆): δ 157.98, 73.77, 70.72. HRMS (ESI): Calcd for [M+H]⁺: 261.1464, Found: 261.1463.

Glycoluril dimer (1.88 g, 6.10 mmol) was weighed and dissolved in methanesulfonic acid (11 mL) under ultrasound. Dimethyl glycoluril diether-*d₆* (4.76 g, 18.3 mmol) was added to the system and the system was stirred mechanically at 50 °C to react for 5 hours. After the reaction was completed, the reaction was cooled to room temperature, the reaction solution was added dropwise to an iced water (110 mL) with generation of a large amount of white precipitate, and the mixture was then centrifuged. The solid was washed in the centrifuge tube with water until neutral. The solid was re-dissolved in trifluoroacetic acid (6 mL) and cooled to 0 °C. Then the solution was slowly added dropwise with water (30 mL), and a large amount of white precipitates generated, which was centrifuged, and washed with water until turning neutral, and freeze-dried to obtain glycoluril tetramer-*d₁₂* as a white solid powder (1.98 g, 41%). ¹H NMR (400 MHz, DMSO-*d₆*): δ 5.69 (d, *J* = 11.0 Hz, 2H), 5.54 (d, *J =* 15.1 Hz, 6H), 5.41 (d, *J =* 9.1 Hz, 2H), 5.16 (d, *J* = 10.9 Hz, 4H), 4.83 (d, *J =* 11.2 Hz, 4H), 4.26 - 4.15 (m, 6H). ¹³C NMR (101 MHz, DMSO-*d₆*): δ 155.18, 154.68, 76.84, 72.12, 70.63, 70.40, 70.02, 52.82, 48.34. HRMS (ESI): Calcd for [M+Na]⁺: 815.3446, Found: 815.3436. Calcd for [M+H]⁺: 793.3627, Found: 793.3619.

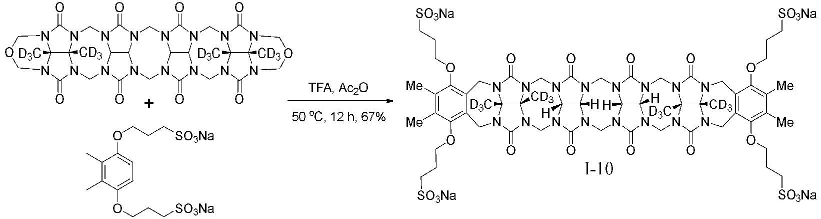

The dimethyl tetramer-*d₁₂* (1.0 g, 1.26 mmol) was weighed and dissolved in trifluoroacetic acid (6 mL). Then acetic anhydride (6 mL) and sodium 2,3-dimethyl-1,4-phenoxydipropylsulfonate (compound 12, 1.6 g, 3.75 mmol) were added in sequence, and then the oil bath temperature was set at 50°C and the reaction system was reacted for 12h. After the reaction was complete, the reaction solution was added dropwise to ethanol (300 mL), stirred for 30 minutes, and then left stand and centrifuged. The solid was repeatedly washed with ethanol in a centrifuge tube (10 mL× 4) until the supernatant was essentially colorless. The solid was transferred to a 50 mL eggplant flask to remove the solvent, and the resulting solid was recrystallized using ethanol and water. After filtration, the solid was washed with a small amount of a mixed solution of ethanol/water (4:1) and dried in vacuum to obtain Compound I-10 (1.36 g, 67%) as a white solid powder. ¹H NMR (400 MHz, D₂O): δ 5.62 (d, *J =* 15.4 Hz, 2H), 5.52 (d, *J =* 15.4 Hz, 4H), 5.46 - 5.34 (m, 4H), 5.10 (d, *J =* 15.9 Hz, 4H), 4.32 (d, *J =* 16.8 Hz, 4H), 4.23 (d, *J =* 15.9 Hz, 4H),4.09 (d, *J =* 15.4 Hz, 2H), 3.91 (s, 4H), 3.69 (s, 4H), 3.11 (s, 8H), 2.18 (s, 8H), 1.81 (s, 12H). ¹³C NMR (101 MHz, D₂O): δ 156.74, 156.15, 150.23, 131.48, 128.22, 78.46, 77.39, 72.79, 71.32, 71.06, 52.60, 48.43, 47.97, 36.21, 24.89, 12.49. HRMS (ESI): Calcd for [M+2Na]²⁺: 827.1959, Found: 827.1950.

### Example 11:

Compound I-7-4H was synthesized according to the method described in Example 7, except that non-deuterated glycoluril tetramer was used instead of tetradeuterated glycoluril tetramer.

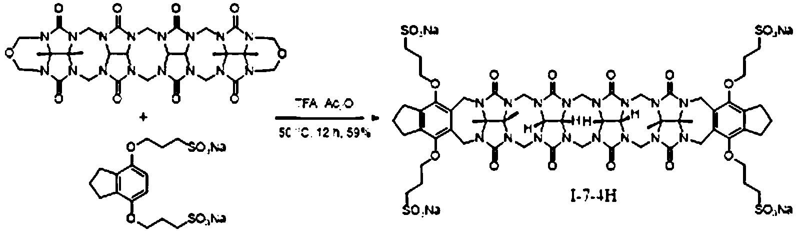

Dimethyl glycoluril tetramer (1.50 g, 1.79 mmol), and trifluoroacetic acid (7.0 mL) were successively added to a 50 mL single-neck flask, and then stirred to dissolve. Then acetic anhydride (7.0 mL) and sodium 2,3-dimethyl-1,4-phenoxydipropylsulfonate (2.35 g, 5.35 mmol) were successively added to the reaction system, and stirred to dissolve. The system was heated to 50 °C and reacted for 12 hours. After the reaction system cooled down, the reaction solution was added dropwise to 140 mL of ethanol to precipitate light yellow solids, which were centrifuged, and collected. The solids were washed twice with 150 mL of ethanol and centrifuged again. The resulting solid was recrystallized using water and ethanol at 70 °C and dried in vacuum to afford a white powdery solid I-7-4H (1.60g, 59%). ¹H NMR (400 MHz, D₂O): δ 5.68 (d, *J =* 15.3 Hz, 2H), 5.56 (d, *J =* 15.7 Hz, 4H), 5.43 (d, *J =* 9.0 Hz, 2H), 5.35 (d, *J =* 9.0 Hz, 2H), 5.18 (d, *J =* 15.9 Hz, 4H), 4.31 (dd, *J =* 59.0, 15.9 Hz, 8H), 4.08 (td, *J =* 11.5, 10.9, 5.7 Hz, 6H), 3.87 (q, *J =* 8.3, 7.5 Hz, 4H), 3.12 (dd, *J =* 20.4, 14.4, 7.2 Hz, 8H), 2.47 (d, *J =* 19.8 Hz, 8H), 2.16 (s, 8H), 1.77 (d, *J =* 19.7 Hz, 12H), 1.43 (s, 2H), 1.31 (s, 2H). ¹³C NMR (101 MHz, D₂O): δ 156.71, 156.19, 148.31, 138.21, 128.65, 78.70, 77.66, 71.88, 71.51, 71.16, 52.93, 48.52, 48.12, 36.08, 30.36, 25.58, 25.03, 16.80, 15.71.

### Example 12

**Water solubility test:** approximately 100 mg of the deuterated compound mentioned above was weighed and added to a graduated thin test tube, and gradually added with deionized water until completely dissolved. The volume was measured to calculate solubility, which was expressed in mg/mL (see Table 1). From the table, it can be seen that although there was a slight difference in solubility, 1-1, I-5, and I-7 had high water solubility and can meet the solubility requirements for rapid injection.

### Solubility (mg/mL)

| medium | I-1 | I-5 | I-7 |
|---|---|---|---|
| deionized water | 510 | 582 | 600 |

### Example 13

**Evaluation of antagonistic activity against muscle relaxants:** taking the reversal of neuromuscular blockade induced by cisatracurium besylate in SD rats by I-1, I-5, and I-7 as an example, the experimental process of reversal of neuromuscular blockade by deuterated diaryl glycoluril tetramer compounds in vivo was explained.

Specific operation process: SD rats weighing 170 to 240 grams were selected as the biological model to test the reversal effect of I-5. Three male and three female mice were selected for each group of experiment (the same applies below). During the testing, the injection solution of neuromuscular blocking agent was prepared using physiological saline as the solvent, and all injection operations were completed within 5 seconds. The rats were anesthetized with isoflurane, with an induction anesthetic dose of 5% and a maintenance anesthetic dose of 1.5%; 10% Na₂S solution was used to remove hair from the right hind leg and nearby area of rats; the airway was inserted into the rat trachea and connected to a small animal ventilator, with a tidal volume set to 8 and a frequency set to 80; a muscle relaxation monitor was used to test the neuromuscular blockade of the quadriceps femoris muscle of rats, wherein, the sensor was fixed to the tibia of the rat, and the electrode was fixed to the right posterior leg of the rat through a patch. Then, the monitor was calibrated in Cal mode and switched to TOF mode (TOF is the abbreviation for train of four, a series of four stimuli) after the data became stable. If the TOF data is stably ≥90, proceed to the next step; cisatracurium besylate solution was administered via the tail vein injection to rats at a dose of 0.6 mg/kg, and the timing was started, and the change in TOF values was recorded; when the TOF value decreased to 0, I-5 solution was injected into rats, and the injection time was recorded. Then, the change in TOF value was recorded, and the time TOF value recovered to ≥ 90 was recorded as the reversal time of neuromuscular blockade.

The antagonistic effects of non-deuterated control compounds I-5-4H and I-7-4H aganist cisatracurium besylate and rocuronium bromide at the same dosage were shown in Tables 2 and 3.

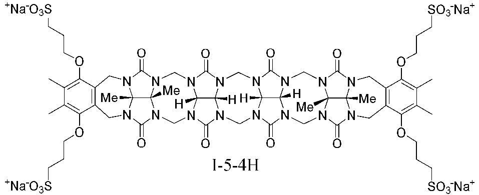

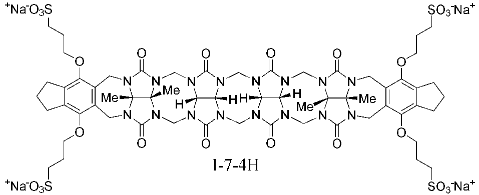

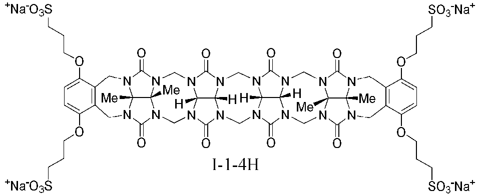

By comparing the antagonistic activity of compounds I-1, I-5, and I-7 with that of the corresponding non-deuterated compounds I-1-4H, I-5-4H, and I-7-4H, it can be found that the deuterated compounds have an enhanced antagonistic activity (see Tables 2 and 3).

At a high dose of 150 mg/kg, the TOF recovery time of non-deuterated control compound I-1-4H for reversing cisatracurium besylate was 84 seconds, and at a dose of 30 mg/kg, the TOF recovery time of I-1-4H for reversing rocuronium bromide was 300 seconds (Hoffmann et al, Anesthesiology 2013, 119, 317-325). Deuterated I-1 respectively reversed cisatracurium besylate and rocuronium bromide at relatively lower doses (80 mg/kg and 25 mg/kg) with significantly improved activity, and the TOF recovery times thereof were 24 seconds and 208 seconds, respectively.

Compared to I-7-4H, the deuterated I-7 exhibited significantly improved reversal activity against cisatracurium besylate and maintained rapid reversal activity against rocuronium bromide; the reversal activity of deuterated I-5 against cisatracurium besylate was significantly improved, achieving rapid reversal against cisatracurium besylate, and maintained rapid reversal activity against rocuronium bromide, and the activity thereof was higher than that of non-deuterated samples at the same dose. Therefore, at the same dose, the antagonistic activity of I-5 was significantly better than that of non-deuterated 1-5-4H.

By comparing the activity of deuterated compounds, it was found that at the same dosage, compound I-5 exhibited excellent antagonistic activity against both cisatracurium besylate and rocuronium bromide with the highest antagonistic activity (see Tables 2 and 3).

When the dose of I-5 was 80 mg/kg, the average times to reverse cisatracurium besylate (see Table 2) and pancuronium bromide (see Table 5) were 19 seconds and 20 seconds, respectively; when the dose of I-5 was 25 mg/kg, the average times to reverse rocuronium bromide (see Table 3) and vecuronium bromide (Table 4) were 9 seconds and 20 seconds, respectively, indicating that I-5 can quickly and efficiently reverse the neuromuscular blockade induced by the above four muscle relaxants in vivo.

Through the above in vivo antagonistic activity experiments in rats, it has been confirmed that I-5 can achieve rapid reversal within 20 seconds. Compared with the commercially available agent neostigmine (at experimental dose of rats converted from the maximum allowable clinical dose), I-5 has increased the efficiency in reversing neuromuscular blockade induced by cisatracurium besylate, rocuronium bromide, vecuronium bromide, and pancuronium bromide by 10-17 times, 18 times, 32 times, and 67 times, respectively; compared with the commercially available agent sugammadex sodium, at the same dose, I-5 can quickly reverse the neuromuscular blockade induced by cisatracurium besylate and pancuronium bromide that cannot be reversed clinically by sugammadex sodium, and the activity thereof in reversing neuromuscular blockade induced by rocuronium bromide and vecuronium bromide was also significantly improved compared to sugammadex sodium, with the time that TOF recovered to 0.9 shortened from 52 seconds and 18 seconds to 18 seconds and 9 seconds, respectively, which was equivalent to an increase of about 1.9 times and 1.0 times in antagonistic activity.

As an example of the composition, at the same dose as deuterated I-5 (80 mg/kg), the composition of deuterated I-5 and non-deuterated I-5-4H (with mass fractions of 50% for each) also exhibited good antagonistic activity, with an antagonistic time of approximately 30 seconds (see Table 6).

The antagonistic data of neostigmine and sugammadex sodium in the table are from literature (Liu, et al, Journal of Medicinal Chemistry, 2022, 65, 16893-16901), all doses are the maximum dose for rats converted from the clinical adult dose (1:6.25).

**Table 2. The TOF→0.9 time of compounds I-C (I-1, I-5, I-7) and I-C-4H (I-5-4H, I-7-4H) at the same dose (80 mg/kg) and neostigmine (0.24 mg/kg) antagonizing cisatracurium besylate.**

| muscle relaxant | Antagonists | Average time (s) |
|---|---|---|
| Cisatracurium Besylate (0.6 mg/kg) | neostigmine | 210 |
| | I-1 | 24 |
| | I-5 | 19 |
| | I-7 | 39 |
| | I-5-4H | 37 |
| | I-7-4H | 65 |

**Table 3. The TOF→0.9 time of compounds I-C (I-1, I-5, I-7), I-C-4H (I-5-4H, I-7-4H) and Sugammadex Sodium at the same dose (25 mg/kg), and neostigmine (0.24 mg/kg) antagonizing rocuronium bromide.**

| Muscle relaxant | Antagonists | Average time(s) |
|---|---|---|
| Rocuronium bromide (3.5 mg/kg) | neostigmine | 363 |
| | Sugammadex Sodium | 52 |
| | I-1 | 208 |
| | I-5 | 18 |
| | I-7 | 23 |
| | I-5-4H | 22 |
| | I-7-4H | 19 |

**Table 4. The TOF→0.9 time of compound I-5 and Sugammadex Sodium at the same dose (25 mg/kg), and neostigmine (0.24 mg/kg) antagonizing vecuronium bromide.**

| Muscle relaxant | Antagonists | Average value (s) |
|---|---|---|
| Vecuronium Bromide (0.7 mg/kg) | neostigmine | 315 |
| | Sugammadex Sodium | 18 |
| | I-5 | 9 |

**Table 5. The TOF→0.9 time of compound I-5 (80 mg/kg), and neostigmine (0.24 mg/kg) antagonizing pancuronium bromide.**

| Muscle relaxant | Antagonists | Average value (s) |
|---|---|---|
| pancuronium bromide (0.5 mg/kg) | neostigmine | 1371 |
| | I-5 | 20 |

**Table 6. The TOF→0.9 time of the composition of compound I-5 and I-5-4H (80 mg/kg, mass fraction of 50% for each) antagonizing cisatracurium besylate.**

| Muscle relaxant | Antagonists | Average value (s) |
|---|---|---|
| Cisatracurium Besylate (0.6 mg/kg) | I-5 (40mg/kg) + I-5-4H (40mg/kg) | 31 |

### Example 14

**Blood pharmacokinetics and excretion:** Deuterated diaryl glycoluril tetramer compounds exert their effects through blood injection. Taking the half-life and excretion of compound I-5 in SD rats as an example, the pharmacokinetic process of deuterated diaryl glycoluril tetramer compounds in vivo was explained.

SD rats (180-220g) were used as experimental models in the present invention, with 3 males and 3 females. Deuterated diaryl glycoluril tetramer compound (80 mg/kg) was injected through the tail vein. Blood samples were collected within 1 hour through jugular vein sampling (total volume 0.4 mL, containing 0.05 mL of 3.8% sodium citrate), and urine was collected within 24 hours through metabolic cages. The analytical procedures for determining plasma drug concentrations were as follows: blood samples were collected at 0 minute, 1 minute, 5 minutes, 10 minutes, 20 minutes, 20 minutes, and 60 minutes, followed by centrifugation at 3000 rpm (4 °C, 10 minutes) and the upper plasma was collected. The plasma was added with 0.25 mL of acetonitrile, shook to precipitate the protein, centrifuged at 10000 rpm for 10 minutes (4 °C) and the supernatant was collected, and evaporated at room temperature to remove acetonitrile as much as possible.

Subsequently, deionized water was added to make up a final volume of 0.5 mL, and the supernatant was collected by centrifugation at 10000 rpm (4 °C, 10 minutes). The blood drug concentration at different times was analyzed and quantified by high-performance liquid chromatography. The analysis and testing process for drug excretion was as follows: after injecting the deuterated diaryl glycoluril tetramer compound (80 mg/kg) into the tail vein, the rats were placed in metabolic cages and fed with food and water normally. Urine was collected every 4 hours for a total of 6-7 times. Urine was centrifuged using an ultrafiltration centrifuge tube (10 KD) to remove urinary protein, and the excretion of compounds through urine within 24 hours was analyzed by high-performance liquid chromatography.

The experimental results showed that the half-life of compound I-5 in the blood concentration of rats was 10 minutes, indicating its rapid elimination or clearance characteristics in vivo. Compound I-5 was mainly excreted in the form of its prototype through urine, with over 70% of the compound excreted within 24 hours, indicating that the compound was characterized by rapid excretion mainly through the kidneys.

All documents mentioned in the present invention are cited as references in this application, just as each document is individually cited as a reference. In addition, it should be understood that, after reading the above teaching content of the present invention, those skilled in the art can make various changes or modifications to the present invention, and these equivalent forms also fall within the scope defined by the appended claims of the present application.

## Claims

1. A deuterated diaryl glycoluril tetramer compound of Formula I, or a pharmaceutically acceptable salt, a hydrate, or a solvate thereof, 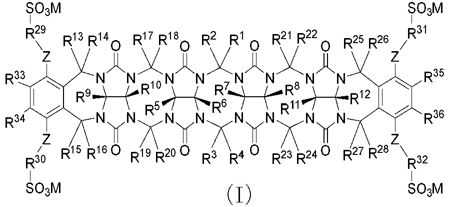 wherein,
each Z is independently O, S, Se, Te, C1-C4 alkylene, -NH- or amino substituted with C1-C4 alkyl;
each M is independently Na⁺, K⁺, Li⁺, Mg²⁺, Ca²⁺, NH₄⁺, or ammonium salts substituted with one or more C1-C4 alkyl;
R¹, R², R³, R⁴, R⁵, R⁶, R⁷, R⁸, R¹³, R¹⁴, R¹⁵, R¹⁶, R¹⁷, R¹⁸, R¹⁹, R²⁰, R²¹, R²², R²³, R²⁴, R²⁵, R²⁶, R²⁷, and R²⁸ are each independently deuterium or hydrogen;
R⁹, R¹⁰, R¹¹, and R¹² are each independently non-deuterated, partially deuterated or fully deuterated linear or branched C1-C20 alkyl, or R⁹ and R¹⁰, R¹¹ and R¹² together with the attached carbon atom each form a non-deuterated, partially deuterated or fully deuterated group selected from the group consisting of: C3-C20 cycloalkyl, 3-20 membered heterocyclyl, C5-C20 aryl, and 5-20 membered heteroaryl;
R²⁹, R³⁰, R³¹, and R³² are each independently non-deuterated, partially deuterated, or fully deuterated groups selected from the group consisting of: linear or branched C1-C20 alkylene, C3-C20 cycloalkylene, linear or branched 1-20 membered heteroalkylene containing one or more heteroatoms selected from oxygen, sulfur, and nitrogen, and 3-20 membered heterocyclylene containing one or more heteroatoms selected from oxygen, sulfur, and nitrogen;
R³³, R³⁴, R³⁵, and R³⁶ are each independently deuterium, hydrogen, or non-deuterated, partially deuterated, or fully deuterated groups selected from the group consisting of: linear or branched C1-C20 alkyl, C3-C20 cycloalkyl, linear or branched C1-C20 alkoxy, linear or branched C1-C20 alkylthio, linear or branched C1-C20 aldehyde group, linear or branched C1-C20 ester group, linear or branched C1-C20 alkyl containing carbonyl, or R³³ and R³⁴, R³⁵ and R³⁶ together with the attached carbon atom each form a non-deuterated, partially deuterated, or fully deuterated group selected from the group consisting of: C3-C20 saturated carbocycle, C3-C20 saturated spiral-ring, 3-20 membered saturated heterocycle containing one or more heteroatoms selected from oxygen, sulfur, and nitrogen, C5-C20 aromatic ring, and 5-20 membered heteroaromatic ring containing one or more heteroatoms selected from oxygen, sulfur, and nitrogen;
with the proviso that: at least one of R¹, R², R³, R⁴, R⁵, R⁶, R⁷, R⁸, R¹³, R¹⁴, R¹⁵, R¹⁶, R¹⁷, R¹⁸, R¹⁹, R²⁰, R²¹, R²², R²³, R²⁴, R²⁵, R²⁶, R²⁷, R²⁸, R³³, R³⁴, R³⁵, and R³⁶ is deuterium, or at least one of R⁹, R¹⁰, R¹¹, R¹², R²⁹, R³⁰, R³¹, R³², R³³, R³⁴, R³⁵, and R³⁶ is deuterium or deuterated.

2. The compound according to claim 1, **characterized in that** the ratio of deuterium atoms to the sum of deuterium atoms and hydrogen atoms (D/(D+H)) in the compound is ≥ 0.015% (natural deuterium isotope content); preferably, ≥ 10%; more preferably, ≥ 30%; more preferably, ≥ 75%; more preferably, ≥ 95%; most preferably, ≥ 98%.

3. The compound according to claim 1, **characterized in that** Z is O, S, Se, Te, methylene, NH or amino substituted with C1-C4 alkyl; R¹, R², R³, and R⁴ are each independently H or D; R⁵, R⁶, R⁷, and R⁸ are each independently hydrogen; R¹⁴, R¹⁵, R¹⁶, R¹⁷, R¹⁸, R¹⁹, R²⁰, R²¹, R²², R²³, and R²⁴ are each independently H or D.

4. The compound according to claim 1, **characterized in that** R⁹, R¹⁰, R¹¹, and R¹² are each independently non-deuterated, partially deuterated or fully deuterated linear or branched C1-C10 alkyl, or R⁹ and R¹⁰, R¹¹ and R¹² together with the attached carbon atom each form a group selected from the group consisting of: C3-C10 cycloalkyl, 3-10 membered heterocyclyl, C6-C10 aryl, and 5-10 membered heteroaryl;
R²⁹, R³⁰, R³¹, and R³² are each independently non-deuterated, partially deuterated, or fully deuterated groups selected from the group consisting of: linear or branched C1-C10 alkylene, C3-C10 cycloalkylene, linear or branched 1-10 membered heteroalkylene containing one or more heteroatoms selected from oxygen, sulfur, and nitrogen, and 3-10 membered heterocyclylene containing one or more heteroatoms selected from oxygen, sulfur, and nitrogen;
R³³, R³⁴, R³⁵, and R³⁶ are each independently deuterium, hydrogen, or non-deuterated, partially deuterated, or fully deuterated groups selected from the group consisting of: linear or branched C1-C10 alkyl, C3-C10 cycloalkyl, linear or branched C1-C10 alkoxy, linear or branched C1-C10 alkylthio, linear or branched C1-C10 aldehyde group, linear or branched C1-C10 ester group, linear or branched C1-C10 alkyl containing carbonyl, or R³³ and R³⁴, R³⁵ and R³⁶ together with the attached carbon atom each form a non-deuterated, partially deuterated, or fully deuterated group selected from the group consisting of: C3-C10 saturated carbocycle, C3-C10 saturated spiral-ring, 3-10 membered saturated heterocycle containing one or more heteroatoms selected from oxygen, sulfur, and nitrogen, C5-C10 aromatic ring, and 5-10 membered heteroaromatic ring containing one or more heteroatoms selected from oxygen, sulfur, and nitrogen.

5. The compound according to claim 1, **characterized in that** R⁹, R¹⁰, R¹¹, and R¹² are each independently non-deuterated, partially deuterated or fully deuterated linear or branched C1-C7 alkyl, or R⁹ and R¹⁰, R¹¹ and R¹² together with the attached carbon atom each form a group selected from the group consisting of: C3-C7 cycloalkyl, 3-7 membered heterocyclyl, C6-C8 aryl, and 5-7 membered heteroaryl;
R²⁹, R³⁰, R³¹, and R³² are each independently non-deuterated, partially deuterated or fully deuterated groups selected from the group consisting of: linear or branched C1-C6 alkylene, and linear or branched 1-6 membered heteroalkylene containing one or more heteroatoms selected from oxygen, sulfur, and nitrogen;
R³³, R³⁴, R³⁵, and R³⁶ are each independently deuterium, hydrogen, or non-deuterated, partially deuterated, or fully deuterated groups selected from the group consisting of: linear or branched C1-C6 alkyl, C3-C7 cycloalkyl, or R³³ and R³⁴, R³⁵ and R³⁶ together with the attached carbon atom each form a non-deuterated, partially deuterated, or fully deuterated group selected from the group consisting of: C3-C7 saturated carbocycle and benzene ring.

6. The compound according to claim 1, **characterized in that** R⁹, R¹⁰, R¹¹, and R¹² are each independently non-deuterated, partially deuterated or fully deuterated linear or branched C1-C4 alkyl, or R⁹ and R¹⁰, R¹¹ and R¹² together with the attached carbon atom each form a group selected from the group consisting of: C5-C7 cycloalkyl;
R²⁹, R³⁰, R³¹, and R³² are each independently non-deuterated, partially deuterated or fully deuterated groups selected from the group consisting of: linear or branched C1-C4 alkylene, and linear or branched 1-4 membered heteroalkylene containing one or more heteroatoms selected from oxygen, sulfur, and nitrogen;
R³³, R³⁴, R³⁵, and R³⁶ are each independently deuterium, hydrogen, CD₃, or CH₃, or R³³ and R³⁴, R³⁵ and R³⁶ together with the attached carbon atom each form a 5-membered carbocycle or benzene ring.

7. The compound according to claim 1, **characterized in that** the compound includes at least one deuterium atom; preferably, 2 deuterium atoms; more preferably, 4 deuterium atoms; more preferably, 6 deuterium atoms; more preferably, 8 deuterium atoms; more preferably, 12 deuterium atoms; most preferably, 20 deuterium atoms.

8. The compound according to claim 1, **characterized in that** R³³, R³⁴, R³⁵, and R³⁶ are each independently deuterium, hydrogen, CD₃, or CH₃, or R³³ and R³⁴, R³⁵ and R³⁶ together with the attached carbon atom each form a 5-membered carbocycle or benzene ring.

9. The compound according to claim 1, **characterized in that** the compound is selected from group consisting of:

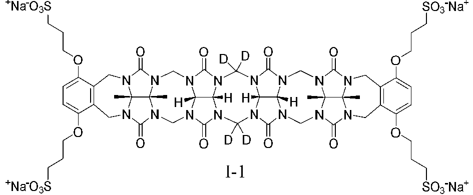

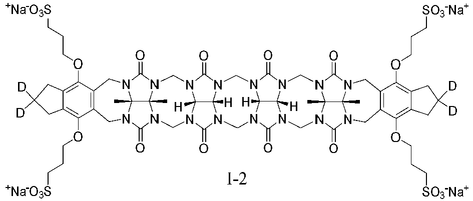

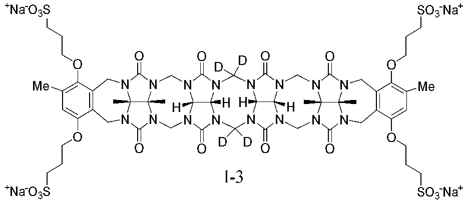

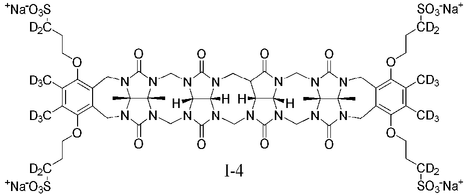

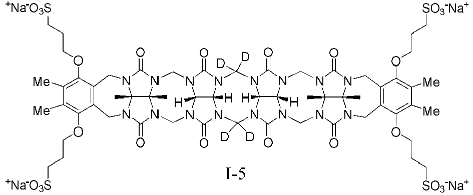

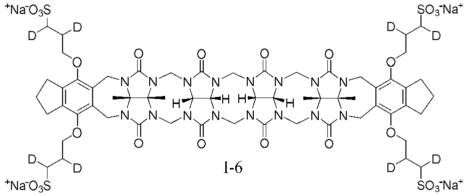

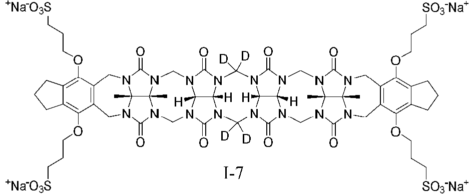

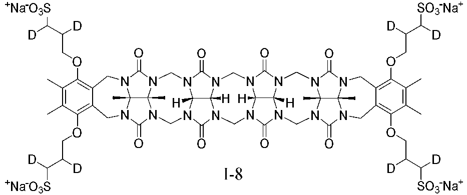

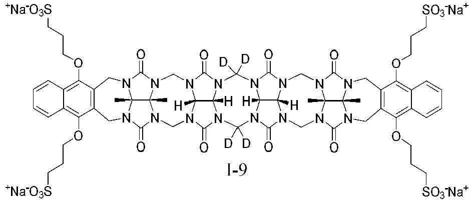

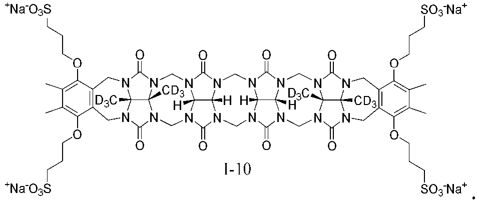

10. The compound according to claim 1, **characterized in that** the compound is

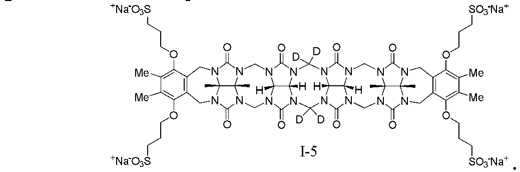

11. A pharmaceutical composition, **characterized in that** comprising: (a) the compound according to claim 1, or the pharmaceutically acceptable salt, the hydrate or the solvate thereof as active ingredient; and (b) pharmaceutically acceptable carriers.

12. A pharmaceutical composition, **characterized in that** comprising:
(a) the compound according to claim 1, or the pharmaceutically acceptable salt, the hydrate or the solvate thereof as a first active ingredient;
(b) components with antagonistic effects on non-depolarizing muscle relaxants as a second active ingredient, and is selected from the group consisting of: Neostigmine, Sugammadex Sodium, or a combination thereof; and
(c) pharmaceutically acceptable carriers or excipients.

13. A pharmaceutical composition, **characterized in that** comprising:
(a) the compound according to claim 1, or the pharmaceutically acceptable salt, the hydrate or the solvate thereof as a first active ingredient;
(b) non-deuterated aryl tetramer compound having the structure shown in claim 1 as a second active ingredient; and
(c) pharmaceutically acceptable carriers or excipients.

14. A kit, **characterized in that** the kit comprising:
(a) a first container, and the compound according to claim 1, or the pharmaceutically acceptable salt, the hydrate, or the solvate thereof, or the pharmaceutical composition according to claim 10 as the active ingredient placed in the first container, and/or
(b) a second container, and a muscle relaxant placed in the second container; the muscle relaxant is selected from the group consisting of: steroid quaternary ammonium salts, tetrahydroisoquinoline quaternary ammonium salts, benzylisoquinoline derivatives, or combinations thereof; and/or
(c) a n-th container, and a n-th drug component placed in the n-th container, wherein n is any positive integer from 3 to 30; wherein, the n-th drug component is the compound according to claim 1; and/or
(4) optional instructions.

15. Use of the compound according to claim 1, the pharmaceutical composition according to claim 11, or the pharmaceutical composition according to claim 12 for antagonizing a muscle relaxant.
